# EUROPEAN PATENT SPECIFICATION

(11) **EP 4 564 370 B1**
(45) Date of publication and mention of the grant of the patent: **04.11.2026**
(21) Application number: 25151041.8
(22) Date of filing: 02.07.2021
(51) Int. Cl.: A61B 5/1473, G16H 50/30

(54) **A DEVICE FOR THE MITIGATION OF A NON-ANALYTE-DERIVED SIGNAL**
VORRICHTUNG ZUR ABSCHWÄCHUNG EINES SIGNALS AUS EINEM NICHT-ANALYTEN
DISPOSITIF POUR L'ATTÉNUATION D'UN SIGNAL DÉRIVÉ D'UN NON-ANALYTE

(30) Priority: 06.07.2020 US 202063048614 P; 08.11.2020 US 202063111057 P
(43) Date of publication of application: 04.06.2025
(62) Document number(s) of the earlier application(s) in accordance with Art. 76 EPC: 21837561.6 / 4 175 546
(73) Proprietor: Biolinq Incorporated, San Diego, CA 92121 (US)
(72) Inventor: WINDMILLER, Joshua, Ray, San Diego, 92121 (US); PEYSER, Thomas, Arnold, San Diego, 92121 (US); CAMPBELL, Alan, San Diego, 92121 (US); SAMANT, Pradnya, Prakash, San Diego, 92121 (US); BHAVARAJU, Naresh, San Diego, 92121 (US); SEDGHAMIZ, Hooman, San Diego, 92121 (US); TANGNEY, Jared Rylan, Encinitas, 92024 (US)
(74) Representative: Cooley (UK) LLP

(56) References cited:
- US-A1- 2009 259 118
- US-A1- 2017 251 958

## Description

### Technical Field

The present invention generally relates to analyte-selective sensors and methods for configuration of the same, and a microneedle applicator integrated internally into a wearable sensor body housing.

### Background Art

The continuous assessment of circulating glucose levels remains of pivotal importance for the management of diabetes mellitus, especially among individuals who require periodic or continuous insulin infusion to manage this chronic condition. This challenge is addressed by the continuous glucose monitor (CGM), which is widely used by individuals with insulin-dependent diabetes mellitus. CGMs were developed throughout the 1990's and first commercialized in 1999 to provide further granularity to guide therapeutic insulin delivery rather than relying solely on infrequent fingerstick capillary blood sampling. In spite of the little-disputed clinical benefit of continuous glucose monitoring over fingerstick capillary blood sampling, CGM adoption among intensively-insulin-managed patients today remains tepid due, in part, to the limited reliability and accuracy of such systems. Indeed, accuracy oftentimes is compromised due to signal perturbations that are both endogenous (sensor warm-up, presence of reactive oxygen species in situ, convection of interstitial fluid) and exogenous (pressure-induced signal attenuation, temperature fluctuations) in nature. In these scenarios, a single sensing element can often be corrupted in its ability to faithfully track dynamical fluctuations in glucose or other circulating analytes of physiologic relevance. However, in line with aims towards improving device accuracy, the integration of both analyte-selective and analyte-invariant sensing modalities as extricable components within a continuous analyte monitor (such as a CGM) is an active area of development. With the above being said, the integration of said sensing elements presents its own set of unique challenges, namely, developing robust methods for the integration of multiple sensing elements into a single transducer, minimizing undue interactions among said sensing elements, and the accurate deposition of unique analyte-selective and analyte-invariant sensing chemistries within the said transducer. In light of these challenges, much of the prior art has instructed of single-transducer designs configured solely for the detection of an analyte. In such embodiments, the analyte sensing system is relegated to multiple electrodes comprised of adjacent metal wires or adjacent metal conduits on a flexible substrate.

Prior art solutions have largely been concerned with the mitigation of non-analyte-related signal perturbations via physical, chemical, algorithmic, and contextual methods. The most pertinent and basic example includes requesting or otherwise prompting the user, through a software interface, to notify the system when partaking in certain activities; these activities include exercise, administration of certain therapeutic agents (i.e. acetaminophen, insulin), or consumption of carbohydrates (of relevance to CGM). Physical methods, as another example, include a reduction in the profile of the body-worn sensing contingent to reduce susceptibility to pressure-induced perturbations of the analyte signal. Within the chemical domain, the synthesis of ever-more selective receptor molecules and diffusive flux-limiting membranes aims to increase sensor selectivity in the wake of the undue influence imparted by the complex array of endogenous (i.e. metabolites, hormones, neurotransmitters, small molecules) and exogenous (i.e. pharmaceuticals, supplements, drugs of abuse) analytes co-habilitating the physiological fluid. Likewise, the implementation of advanced signal processing algorithms targeted at outlier detection, fault compensation, and non-physiological rates of change are often employed to reduce the preponderance of signal artifacts and the deleterious contribution of various physical and chemical processes upon sensor performance. More recently, sensor fusion methods aimed at contextual assessment of the user and the state of their body-worn sensor, have been studied as potential countermeasures to reducing the preponderance of signal artifacts. In such solutions, data from orthogonal measures (kinesthetic, electrophysiological, electrodermal, optophysiological sensors, among others) is integrated in a fusion algorithm in order to better understand the likelihood of non-analyte-derived contributions to the signal transduced by the analyte-selective sensor.

Microneedle arrays (MNAs) require insertion within a specific velocity range. Usually the MNAs are a component of a mechanically rigid assembly including the electronics, housing, adhesive, and MNA mounted into a sensor body. Inserting this entire sensor body presents a number of challenges including accelerating the mass to speed with a force over a short distance, stretching the skin prior to accelerating the sensor body into the skin so that the skin will be tight and not displace away from the MNA on impact, releasing the sensor body subsequently from the applicator, along with a myriad of other concerns such as the total cost and size of the applicator and preventing unintended misuse of this complex multi-stage mechanism.

Stretching the skin causes discomfort to the user and the impact of the sensor on the skin also causes discomfort to the user.

This same impact of the sensor body traveling at relatively high speeds can also cause the human body to react with immunological reactions such as redness, swelling, erythema, and/or edema in the area where the sensor was installed. This reaction elongates sensor warm up times and causes increased user discomfort and distress.

Existing needle-, trocar-, and cannula-based analyte-sensors, configured for the selective detection of a target analyte (i.e. glucose) are also sensitive to external mechanical, electrical, and chemical stimuli that corrupt the accurate determination of the target analyte or plurality of analytes. Specifically, these stimuli are largely manifested in undesired perturbations of the signal or signals transduced from said analyte-selective sensors, which serves to introduce error into measurement and thereby undermines the ultimate accuracy achievable with such devices. Indeed, these errors often compound and can result in potentially life-threatening circumstances when the analyte determination tendered by an analyte-selective sensor is utilized in a closed-loop system configured to manage chronic disease. In such a scenario, a potentially lethal dose of therapeutic agent can be delivered, autonomously and sans user intervention, as a response to counteract a perceived pathophysiological reading; a pertinent example is within the domain of automated insulin delivery (AID) for individuals with intensive insulin-managed diabetes. Although analyte-selective sensors operate in a manner to enhance the selectivity towards a target analyte via the implementation of a receptor molecule (i.e. enzyme, antibody, aptamer), capture probe (i.e. single-stranded DNA), or selective catalyst (i.e. noble metal, inorganic species, electrochemical mediator) these devices nevertheless often succumb to exogenous influences that include pressure-induced signal attenuations, non-specific binding of extraneous analytes to the receptor molecule, changes in equilibrium conditions, and interactions with endogenous and exogenous chemical species occupying the physiological milieu, among a number of others. This is largely due to challenges associated with the inability to extricate or otherwise measure the contributions imparted by external stimuli, which are largely non-deterministic and stochastic in nature. To address this challenge, analyte sensing systems have been constructed featuring both analyte-selective and analyte-invariant sensing elements, each embodying a unique chemical constituency, in order to ratiometrically scale the analyte-selective sensor response to mitigate external sources of undue signal influence. However, in practice, noteworthy difficulties arise when attempting to deposit dissimilar chemistries on sensor geometries wherein both analyte-selective and analyte-invariant sensors are co-located within a single aggregated sensing element / transducer or intermingled in close proximity, which can lead to undesirable effects such as cross-talk. More recent efforts have been targeted at algorithmic and contextual methods of de-noising the analyte signal without requiring the addition of a second sensing modality, albeit these approaches have enjoyed very limited success.
US 2009/259118 A1 discloses methods and devices to detect analyte in body fluid. Embodiments include analyte sensors designed so that at least a portion of the sensor is positionable beneath a skin surface in the dermal layer.

### Summary Of The Disclosure

The ability to identify signal contributions which are non-analyte in origin enables the implementation of various mathematical methods to deconvolve or otherwise extricate the signal that is purely analyte-derived in origin from the signal arising from external influences. The current disclosure instructs of the implementation of at least two distinct sensing elements residing within a microneedle array, whereby at least one unique sensing element embodies the ability to quantify the presence of a target analyte (analyte-selective sensor) and is otherwise sensitive, albeit undesirably, to external stimuli and at least one unique sensing element which is not selective towards the presence of a target analyte (analyte-invariant sensor) and is otherwise sensitive, desirably, to external stimuli.

The current disclosure instructs of devices and methods to mitigate the erroneous signal imparted by physical and/or chemical process incident upon analyte-selective electrochemical sensors that are non-analyte-related in origin. These processes often serve to corrupt the measurement signal tendered by said analyte-selective sensors. The solution described herein concerns the implementation of an analyte-invariant measure that is otherwise sensitive to physical and chemical perturbations incident upon the sensing system. This requires the construction of a sensing system featuring at least one of an analyte-selective sensor and at least one of an analyte-invariant sensor. In the preferred embodiment, the analyte-invariant sensor exhibits identical construction and constituency as the analyte-selective sensor sans the addition of an active biorecognition element, affinity molecule, catalyst, or capture probe that is selective towards the target analyte. In an alternative embodiment, a deactivated biorecognition element, expressing no residual biospecific activity, may be included in the analyte-invariant sensor. In yet another embodiment, the said active biorecognition element may be incorporated in the analyte-invariant sensor, but is subject to a deactivation process during sensor manufacture. In this fashion, any non-analyte signal perturbations will be incident upon both the analyte-selective and analyte-invariant sensing elements and can, through various mathematical transformations, be extricated from the fundamental analyte-derived signal in order to maximize the accuracy and reliability of the measurement. In this manner, the mitigation of common-mode influences upon the analyte-selective sensor, which are also detected by the analyte-invariant sensor, can be achieved and hence an overall improvement to the analyte signal fidelity (e.g., signal-to-noise ratio or similar characteristic) can be expected.

Another objective is to eliminate the need to stretch the skin to insert a MNA.

Anther objective is the ability to insert a wide variety of needles, dull or sharp, effectively.

One aspect of the present disclosure is a device for the mitigation of a non-analyte-derived signal perturbation incident upon a body-worn, microneedle array-based analyte sensor. The device comprises a first electrode and a second electrode. The first electrode is positioned on a surface of a first microneedle of a microneedle array. A selective recognition element is disposed on the first electrode and configured to generate a product or change in physical state arising from the interaction of the selective recognition element and an analyte. A membrane is disposed on the selective recognition element. The second electrode is positioned on a surface of a second microneedle of the microneedle array, and a membrane is disposed on the second electrode. The first electrode and second electrode are positioned in spatially distinct locations within a viable epidermis or dermis of a user. A bias potential or current is applied to each of the first electrode and the second electrode. An ensuing electrical response from each of the first electrode and the second electrode is measured. A mathematical transformation is applied to the electrical response generated at the first electrode as a function of the electrical response generated at the second electrode to cause an attenuation of the common-mode signal.

Another aspect of the present disclosure is a device for the mitigation of a non-analyte-derived signal perturbation incident upon a body-worn, analyte sensor. The device comprises a first electrode and a second electrode. A selective recognition element is disposed on the first electrode and configured to generate a product arising from the interaction of the selective recognition element and an analyte. A membrane is disposed on the selective recognition element. A membrane is disposed on the second electrode. The first electrode and second electrode are positioned in spatially distinct locations within a viable epidermis or dermis of a user. A bias potential or current is applied to each of the first electrode and the second electrode. An ensuing electrical response from each of the first electrode and the second electrode is measured. A mathematical transformation is applied to the electrical response generated at the first electrode as a function of the electrical response generated at the second electrode to cause an attenuation of the common-mode signal.

Yet another aspect of the present disclosure is a device, with an analyte-selective sensor and an analyte-invariant sensor, for the mitigation of a non-analyte-derived signal perturbation incident upon a body-worn, analyte sensor. The device comprises an analyte-selective sensor and an analyte-invariant sensor. The analyte-selective sensor comprises a first electrode and a selective recognition element disposed on the first electrode and configured to generate a product or change in physical state arising from the interaction of the selective recognition element and an analyte. A membrane is disposed on the selective recognition element. An analyte-invariant sensor comprises a second electrode and a membrane disposed on the electrode. The analyte-selective sensor and the analyte-invariant sensor are positioned in spatially distinct locations within a viable epidermis or dermis of a user. A bias potential or current is applied to each of the analyte-selective sensor and the analyte-invariant sensor. An ensuing electrical response is measured from each of the analyte-selective sensor and the analyte-invariant sensor. A mathematical transformation is applied to the electrical response generated at the analyte-selective sensor as a function of the electrical response generated at the analyte-invariant sensor to cause an attenuation of the common-mode signal.

Yet another aspect of the present disclosure is a method for the mitigation of a non-analyte-derived signal perturbation incident upon a body-worn, microneedle array-based analyte sensor. The method includes positioning a first microneedle and a second microneedle of a microneedle array in spatially distinct locations within the viable epidermis or dermis of a user, wherein said first microneedle features a first electrode, a selective recognition element disposed on said first electrode and configured to generate a product or change in physical state arising from the interaction of said selective recognition element and the analyte, and a membrane is disposed on the selective recognition element and the second microneedle features a second electrode and a membrane disposed on the second electrode. The method also includes applying a bias potential or current to each of the first electrode and the second electrode. The method also includes measuring an ensuing electrical response from each of the first electrode and second electrode. The method also includes applying a mathematical transformation to the said electrical response generated at the first electrode as a function of the said electrical response generated at the second electrode to cause an attenuation of the common-mode signal.

Yet another aspect of the present disclosure is a method for the mitigation of a non-analyte-derived signal perturbation incident upon a body-worn, analyte sensor. The method includes positioning a first electrode and a second electrode of an analyte sensor in spatially distinct locations within the viable epidermis or dermis of a user, wherein the first electrode comprises a selective recognition element disposed on the first electrode and configured to generate a product or change in physical state arising from the interaction of the selective recognition element and the analyte, and a membrane is disposed on the selective recognition element and the second electrode comprises a membrane disposed on the second electrode. The method also includes applying a bias potential or current to each of the first electrode and the second electrode. The method also includes measuring an ensuing electrical response from each of the first electrode and the second electrode. The method also includes applying a mathematical transformation to the electrical response generated at the first electrode as a function of the electrical response generated at the second electrode to cause an attenuation of the common-mode signal.

Yet another aspect of the present disclosure is a method for the mitigation of a non-analyte-derived signal perturbation incident upon a body-worn, analyte sensor system. The method includes positioning an analyte-selective sensor and analyte-invariant sensor of said analyte sensor system in spatially distinct locations within the viable epidermis or dermis of a user, wherein the analyte-selective sensor features a first electrode, a selective recognition element disposed on the first electrode and configured to generate a product or change in physical state arising from the interaction of the selective recognition element and the analyte, and a membrane disposed on the selective recognition element and the analyte-invariant sensor comprises a second electrode and a membrane disposed on the second electrode. The method also includes applying a bias potential or current to each of said analyte-selective sensor and analyte-invariant sensor. The method also includes measuring an ensuing electrical response from each of the analyte-selective sensor and the analyte-invariant sensor. The method also includes applying a mathematical transformation to the electrical response generated at the analyte-selective sensor as a function of the electrical response generated at the analyte-invariant sensor to cause an attenuation of the common-mode signal.

The analyte preferably includes at least one of a biomarker, chemical, biochemical, metabolite, electrolyte, ion, hormone, neurotransmitter, vitamin, mineral, drug, therapeutic, toxin, pathogen, infectious agent, allergen, enzyme, protein, nucleic acid, DNA, and RNA.

The analyte sensor system is preferably a microneedle or a microneedle array, with each microneedle constituent possessing a vertical extent preferably between 200 and 2000 µm.

The microneedle or microneedle array preferably contains at least one projection capable of insertion into the viable epidermis or dermis of a user.

The first electrode and the second electrode preferably include a metal, metal alloy, metal oxide, semiconductor, or polymeric surface.

The first electrode and the second electrode are confined to the tapered distal region of the microneedle or the elements of the microneedle array.

The selective recognition element preferably includes at least one of an enzyme, aptamer, antibody, capture probe, ionophore, catalyst, biocatalyst, DNA, RNA, organelle, or cell.

The product is preferably a chemical, biochemical, mediator, resistance change, electrical signal, electrochemical signal, conductance change, impedance change, or absorbance change.

The membrane is preferably at least one of a polymer, hydrophilic layer, biocompatible layer, diffusion-limiting layer, hydrogel, film, and coating.

The bias potential or current is preferably either of the direct current or alternating current variety.

The electrical response preferably includes at least one of a potential, current, impedance, conductance, resistance, capacitance, and inductance.

The mathematical transformation preferably includes at least one of a difference operation, denoising operation, regression, deconvolution, Fourier decomposition, background subtraction, Kalman filtering, and Maximum Likelihood Estimation.

The attenuation preferably includes at least one of the removal, minimization, or reduction in duration of the common-mode signal.

The common-mode signal preferably includes at least one of a warm-up signal following application of the microneedle array-based analyte sensor to the skin of a wearer, a pressure-induced signal artefact, a temperature-induced signal fluctuation, and an interference signal originating from an endogenous or exogenous chemical species circulating in a physiological fluid of a user.

The endogenous or exogenous chemical species preferably includes at least one of a biomarker, chemical, biochemical, metabolite, electrolyte, ion, hormone, neurotransmitter, vitamin, mineral, drug, therapeutic, toxin, pathogen, infectious agent, allergen, enzyme, protein, nucleic acid, DNA, and RNA.

The physiological fluid is preferably at least one of interstitial fluid, dermal interstitial fluid, or blood of a user.

### Brief Description Of The Drawings

FIG. 1 is a block diagram illustrating the use of an analyte-invariant signal in conjunction with an analyte-selective signal to de-noise the analyte signal.
FIG. 2 is raw signal traces originating from an analyte-invariant sensor and an analyte-selective sensor.
FIG. 3 is a raw signal traces originating from a plurality of analyte-selective sensors.
FIG. 4 is a bar chart illustrating improvements to analyte-selective sensor accuracy.
FIG. 5 is a diagram of a prior art analyte-selective sensor block / process-flow diagram. The non-analyte signal is additive to the analyte signal.
FIG. 6 is a block / process-flow diagram of an analyte-invariant sensor.
FIG. 7 is a block / process-flow diagram of a system to remove the perturbations to the analyte signal that are non-analyte (and additive) in origin.
FIG. 8 is a block / process-flow diagram of a system to remove the perturbations to the analyte signal that are non-analyte (and additive) in origin.
FIG. 9 is a block / process-flow diagram of a system to remove the common-mode signal arising from perturbations that are non-analyte in origin.
FIG. 10 is a block / process-flow diagram of a system to remove the common-mode signal arising from perturbations that are non-analyte in origin.
FIG. 11 is a block diagram of a device to remove signal perturbations that are non-analyte in origin.
FIG. 12 is a flow chart of a method of the disclosure under the microneedle embodiment.
FIG. 13 is a flow chart of a method of the disclosure under the electrode embodiment.
FIG. 14 is a flow chart of a method of the disclosure under the analyte-selective and analyte-invariant embodiment.

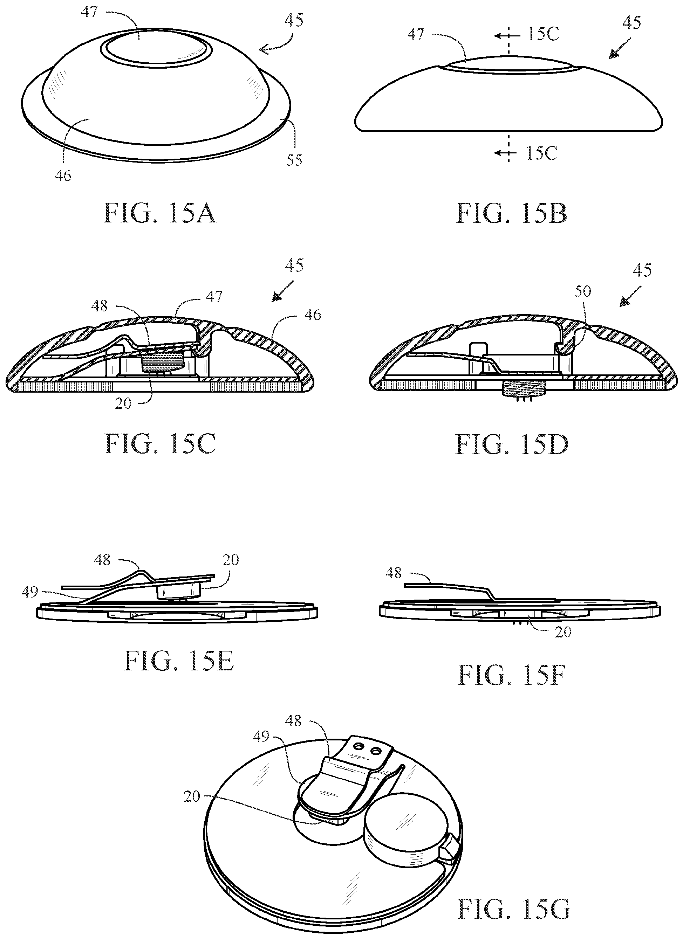

FIG. 15A is a top plan view of an embodiment of the device of the disclosure.
FIG. 15B is a side view of an embodiment of the device of the disclosure.
FIG. 15C is sectional view of the device of FIG. 15B with the MNA retracted.
FIG. 15D is sectional view of the device of FIG. 15B with the MNA released.
FIG. 15E is a side view of an embodiment of the device of the disclosure with the housing removed, with the MNA retracted.
FIG. 15F is a side view of an embodiment of the device of the disclosure with the housing removed, with the MNA released.
FIG. 15G is a perspective view of the device of FIG. 15C.
FIG. 16 an illustration of a cross-section of skin with subcutaneously-implanted microneedles with electrodes.
FIG. 17A is an illustration of a microneedle array configured with a first electrode with a selective recognition element disposed on the first electrode, a membrane disposed on the selective recognition element, and a membrane disposed on the second electrode.
FIG. 17B is an illustration of a microneedle array configured with a first electrode with a selective recognition element disposed on the first electrode, and a membrane (blanket disposition).
FIG. 17C is an illustration of a microneedle array configured with a first electrode with a membrane disposed containing a selective recognition element on the first electrode, and a membrane disposed on the second electrode.
FIG. 17D is an illustration of a microneedle array illustrating the major components and measurements.
FIG. 18A a top plan view of an embodiment of the device of the disclosure.
FIG. 18B is a side view of the device of FIG. 18A.
FIG. 18C illustrates an exploded view rendering of the device of FIG. 18A.
FIG. 19 illustrates electronic circuitry contained in prototype wearable device enclosure designed to interface directly with a microneedle-based biosensor device.
FIG. 20 illustrates another view of the electronic circuitry contained in prototype wearable device enclosure designed to interface directly with a microneedle-based biosensor device.
FIG. 21 illustrates electronic circuitry contained in sealed housing with access to the microneedle device provided via gold-plated pressure connectors located on the viewable surface of the housing.
FIG. 22 illustrates a skin-penetrating hollow microneedle array comprising a plurality of protrusions having vertical extent of approximately 1000 µm, with each element of the microneedle array functionalized to impart selective biosensing ability.
FIG. 23A illustrates a hollow, unfunctionalized microneedle array.
FIG. 23B illustrates a hollow "filled", functionalized microneedle array with selective biosensing ability.
FIG. 24 illustrates an exploded view rendering of complete microneedle biosensing system illustrating all functional components, including the microneedle biosensor and printed circuit board containing the electronic circuitry required to transduce biochemical signals to digital data that can be wirelessly transmitted to an external device via an embedded wireless transceiver.
FIG. 24A is an isolated enlarged view of the microneedle biosensor component of FIG. 24.
FIG. 25 illustrates another view of the wearable microneedle biosensing system.
FIG. 26 illustrates a posterior surface view of the electronics components.
FIG. 27 illustrates a detailed block/process flow diagram.
FIG. 28 is a circuit diagram of a standalone potentiostat integrated circuit.
FIG. 29 is a circuit diagram of a multi-component potentiostat.
FIG. 30 is a block diagram of a difference amplifier.
FIG. 31 is a signal flow diagram of the present disclosure.
FIG. 32 is a circuit diagram of an integrated analog front end and sensor interface.
FIG. 33 is a circuit diagram of mirrored difference amplifiers and filtering.
FIG. 34 is a circuit diagram of fixed mirrored instrumentation amplifiers.
FIG. 35 is a circuit diagram of digital potentiometer-adjustable mirrored instrumentation amplifiers.
FIG. 36 is an illustration of a handheld analyzer in a large form factor.
FIG. 37 is an illustration of a handheld analyzer in a small form factor.
FIG. 38 is a block diagram of a sample algorithm.
FIG. 39 is an illustration of a handheld analyzer in a small form factor.

### Best Mode(s) For Carrying Out The Invention

Body-worn analyte-selective sensors, such as continuous glucose monitors, are sensitive electrochemical systems that are configured to sense an analyte, or plurality of analytes, in a selective fashion with a high-degree of accuracy. This accuracy can be unduly influenced by various external stimuli, which gives rise to undesired perturbations of the signal or signals transduced from said analyte-selective sensors, thereby introducing error in measurement and undermining the ultimate accuracy achievable with such devices. In this vein, even the most proficient analyte-selective sensors often succumb to the influence of external perturbations, which may be chemical, electrical, or mechanical in origin. The current innovation is aimed at mitigating the preponderance of undue physical, chemical, and otherwise exogenous influences upon the fidelity of the measurement of the target analyte or plurality of analytes. This is achieved via implementation of at least one of an analyte-selective sensor and at least one of an analyte-invariant sensor, whereby the said analyte-selective sensor features a selective recognition element and said analyte-invariant sensor lacks said selective recognition element but is otherwise identical in construction and constituency to said analyte-selective sensor. Using a mathematical transformation, algorithm, or combination thereof, the common-mode signal appearing at both the analyte-selective and analyte-invariant sensors may be minimized, mitigated, or eliminated entirely, thereby resulting in an analyte signal of greater fidelity and/or accuracy.

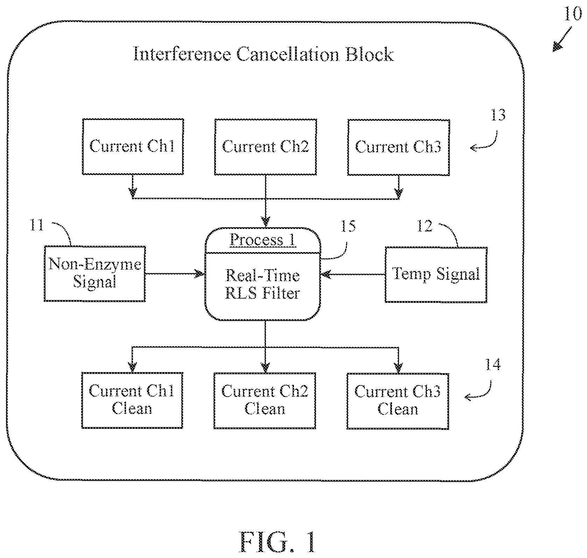

FIG. 1 shows a block diagram 10 illustrating the use of an analyte-invariant (Non-Enzyme) signal 11 in conjunction with an analyte-selective (Current Ch1, Ch2, Ch3) signal 13 to de-noise the analyte signal 14 (Current Ch1 Clean, Ch2 Clean, Ch3 Clean). ***What about Temp signal 12? Process 1* /*RLS filter 15?*

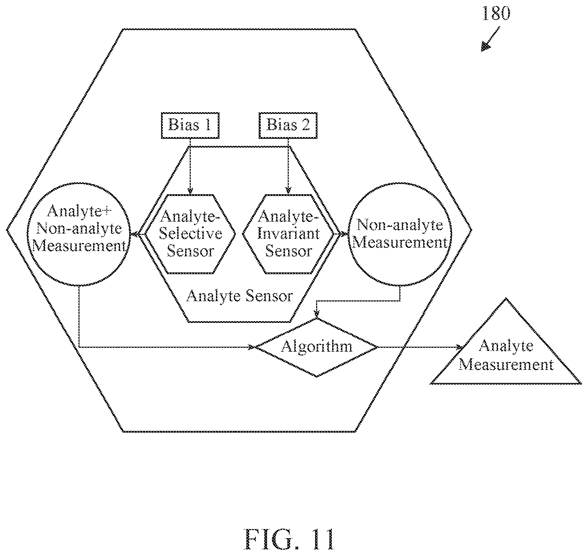

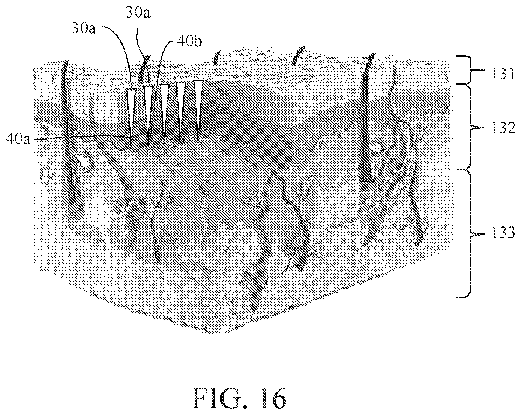

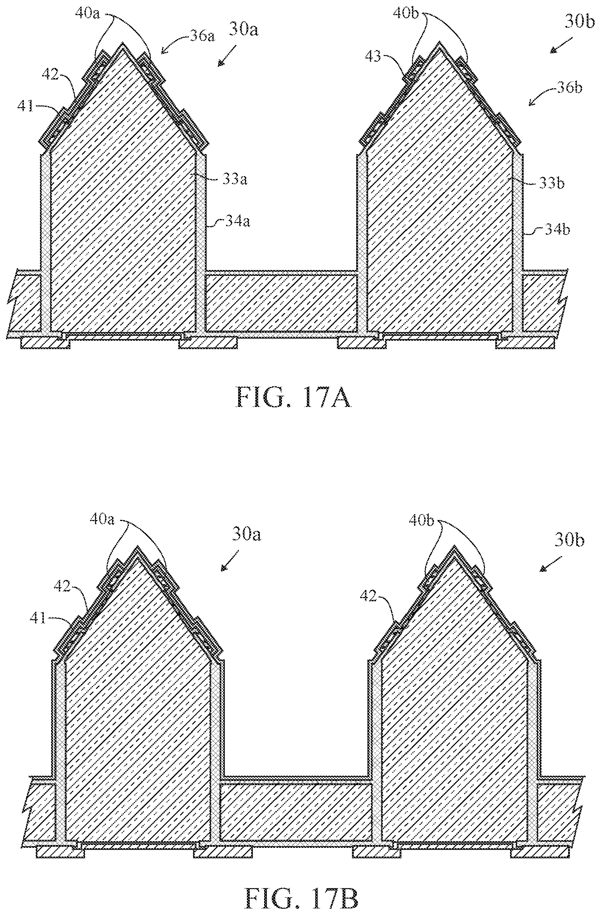

In order to mitigate non-analyte-derived signal perturbations incident upon a body-worn, microneedle array-based analyte sensor, the said device is configured to feature at least one of an analyte-selective sensor and at least one of an analyte-invariant sensor, both located on unique microneedle constituents of the array, as show in FIG. 17A. Specifically, said analyte-selective sensor is configured to feature an electrode 40a on the surface of a first microneedle 30a of said microneedle array, a selective recognition element 41 disposed on said first electrode 40a and configured to generate a product arising from the interaction of said selective recognition element 41 and said analyte, and a membrane disposed 42 on said selective recognition element 41. Likewise, said analyte-invariant sensor is configured to feature an electrode 40b on the surface of a second microneedle 30b of said microneedle array, and a membrane 43 disposed on said electrode. Said analyte-selective and analyte-invariant sensors are disposed in said microneedle array to facilitate sensing operation in spatially distinct locations within the viable epidermis 131 or dermis 132 of a user, as shown in FIG. 16, thereby serving to minimize any undue influence or cross-talk from one sensor to another. Upon the application of an identical or unique bias signal (DC or AC potential or current), as shown in FIG. 11, to both the analyte-selective and analyte-invariant sensors, an ensuing electrical response (potential, current, impedance, conductance, resistance, capacitance, or inductance) is measured from both the said first electrode and second electrode. A mathematical transformation is subsequently applied to the said electrical response generated at the first electrode as a function of the said electrical response generated at the second electrode to remove the common-mode signal incident upon both analyte-selective and analyte-invariant sensors. These mathematical transformations can include differential (subtractive) measurement, deconvolution, Fourier decomposition, background subtraction, Kalman filtering, and Maximum Likelihood Estimation.

In another embodiment of the present disclosure, as shown in FIG. 17B, the analyte-selective sensor is configured to feature an electrode 40a on the surface of a first microneedle 30a of the microneedle array, a selective recognition element 41 disposed on the first electrode 40a and a membrane disposed 42 on the selective recognition element 41 and on the second electrode 40b of the second microneedle 30b.

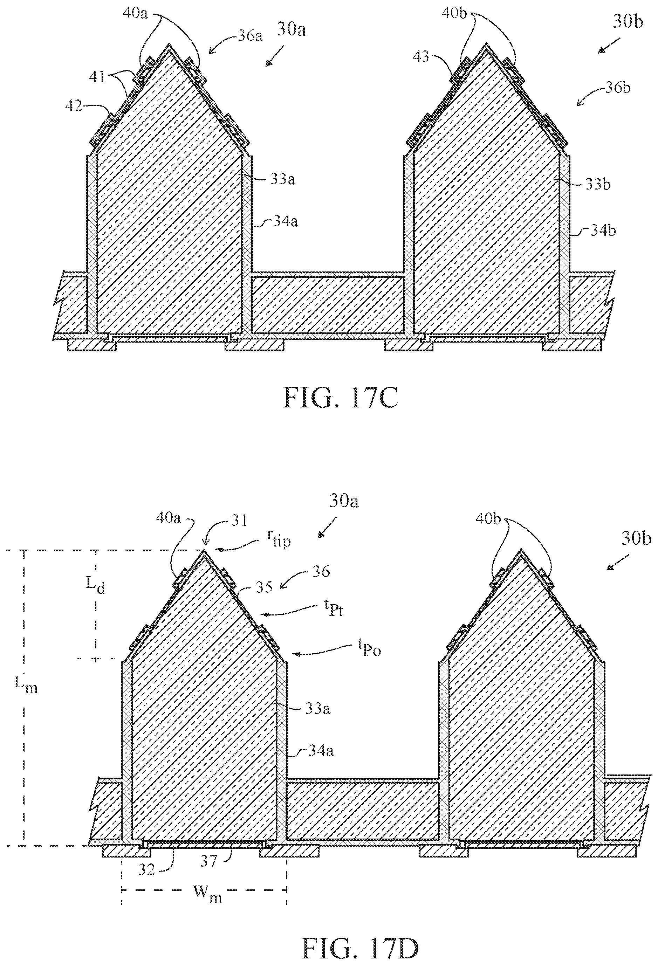

In yet another embodiment of the present disclosure, as shown in FIG. 17C, the sensor is configured to feature an electrode 40a on the surface of a first microneedle 30a of the microneedle array, and a membrane 42 containing a selective recognition element 41 disposed on the first electrode 40a. A membrane 43 is disposed on the second electrode 40b on the surface of a second microneedle 30b.

As shown in FIG. 17D, each microneedle 30 of the microneedle array 20 preferably has a through-silicon via 33 embedded within a microneedle 30. The microneedle 30 preferably has insulation 34 composed of an oxide. This allows the sensors to be individually probed as isolated constituents of the microneedle array 20. The microneedle array preferably can be reflow-soldered to nearly any circuit board just like an integrated circuit. Each microneedle 30 preferably has an individual sensor 31 confined to a distal tip of the microneedle 30, preferably in a region between 1 and 1500 µm from the distal end of the microneedle 30. The microneedle 30 preferably has a backside metal contact 32, a through needle VIA 33, insulation 34 to electrically isolate the microneedle 30 and a patterned metal contact 35 on the distal tip 36 of the microneedle 30. The backside metal contact 32 is preferably composed of a nickel/gold material with an interior portion 37 composed of an aluminum material. The microneedle 30 preferably has a through needle VIA 33 composed of a silicon material. The distal tip 36 preferably has oxide portions and platinum portions. The length, Lm, of the microneedle 30 preferably ranges from 200-2000 µm, and is most preferably 625 µm. The width, Wm, of the microneedle 30 preferably ranges from 100 to 500 µm, and is most preferably 160 µm. The distal tip 36 preferably has a length, Ld, ranging from 50 to 200 µm, and is most preferably 100 µm.

The devices and methods presented are capable of the determination of analytes that comprise at least one of a biomarker, chemical, biochemical, metabolite, electrolyte, ion, hormone, neurotransmitter, vitamin, mineral drug, therapeutic, toxin, enzyme, protein, nucleic acid, aptamer, DNA, and RNA. Furthermore, these systems employ microneedle arrays containing at least two projections capable of insertion into the viable epidermis or dermis of a user, wherein each projection possesses an extent between 200 and 2000 micrometers from proximal to distal extremities. The electrode constituent discussed above is confined to the distal region of the aforementioned protrusions and includes a metal, semiconductor, or polymeric surface. The selective recognition element discussed includes at least one of an enzyme, aptamer, antibody, capture probe, ionophore, catalyst, biocatalyst, DNA, RNA, organelle, or cell and is configured to produce a chemical, biochemical, mediator, resistance change, electrical signal, conductance change, impedance change, or absorbance change upon exposure to the analyte. The abovementioned membrane is at least one of a polymer, hydrophilic layer, biocompatible layer, diffusion-limiting layer, hydrogel, film, and coating.

Other novel and utilitarian features of the disclosure includes its intrinsic ability to negate the effect of cross-talk due to diffusive transport of product from analyte-selective to analyte-invariant sensor. The disclosure also reduces the influence of the analyte depletion region or diffusion layer effects, which serves to limit the quantity of analyte that can diffuse to an analyte-selective sensor.

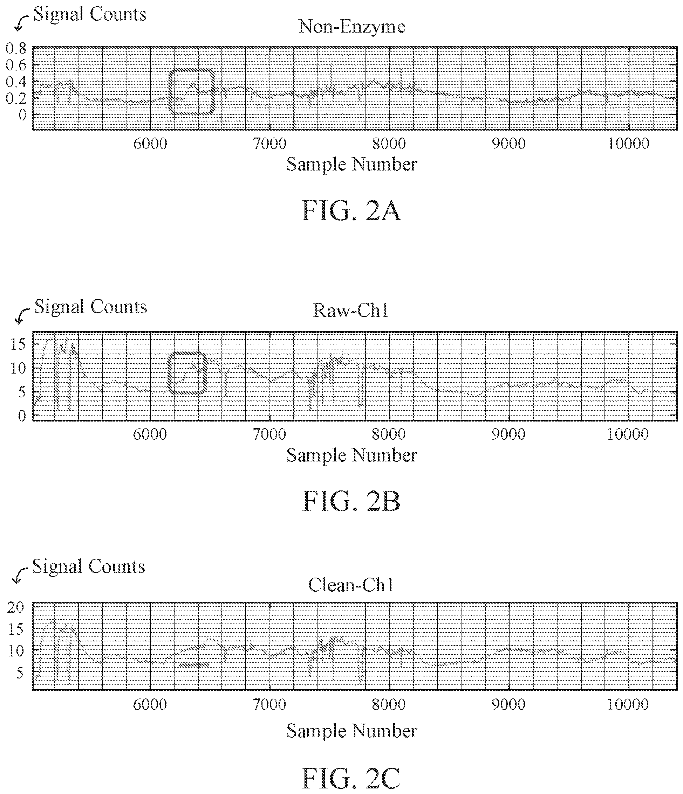

FIGS. 2A-2C show raw signal traces originating from an analyte-invariant sensor (Non-Enzyme), as shown in FIG 2A, and an analyte-selective sensor (Raw-Ch 1), as shown in FIG. 2B, illustrating the common-mode signal perturbation highlighted in the red square. The implementation of a mathematical transformation allows the removal of the said common-mode signal perturbation (red line), as shown in FIG. 2C, which is non-analyte in origin as it appears at both the analyte-invariant and analyte-selective sensors.

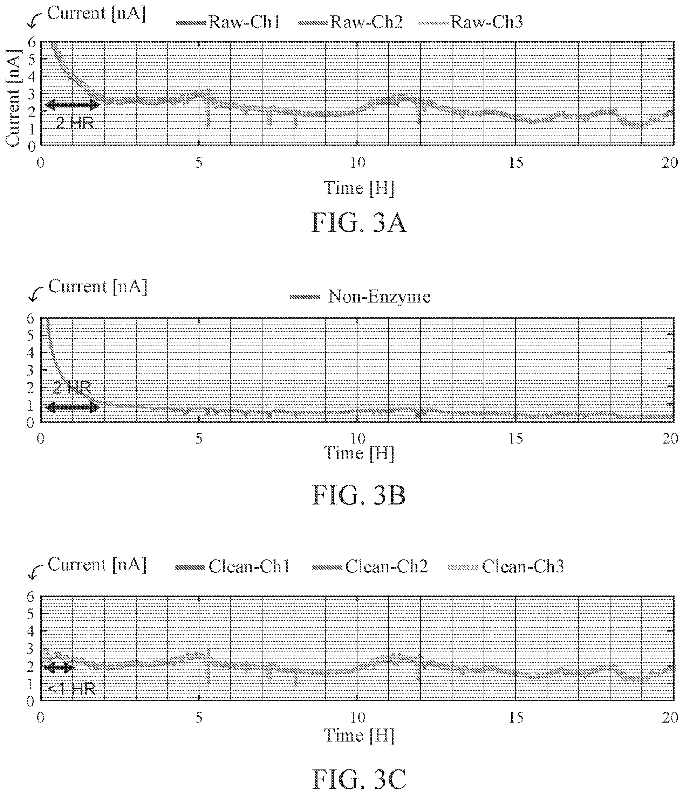

FIGS. 3A-3C show raw signal traces originating from a plurality of analyte-selective sensors (Raw-Ch1, Ch2, Ch3), as shown in FIG. 3A, and an analyte-invariant sensor (Non-Enzyme), as shown in FIG. 3B, illustrating the 2 hour warm-up period required for sensor equilibration following implantation into tissue. The implementation of a mathematical transformation allows the reduction of the apparent warm-up period to less than 1 hour, as shown in FIG. 3C. The warm-up period is non-analyte in origin as it appears at both the analyte-invariant and analyte-selective sensors.

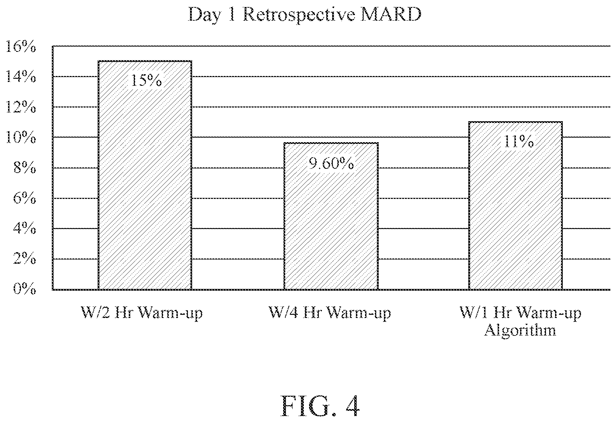

FIG. 4 shows a bar chart illustrating improvements to analyte-selective sensor accuracy (as evidence by mean absolute relative difference - MARD) on day 1 of sensor use by means of extending the warm-up period or implementation of an algorithm configured to subtract the analyte-invariant sensor signal from the analyte-selective sensor signal.

Assuming that the analyte-selective sensor is sensitive to non-analyte signal perturbations (in addition to the analyte signal) and that the analyte-invariant sensor is purely a function of the non-analyte signal perturbation (i.e. not influenced by the analyte signal), the true analyte signal is isolated by differential measurement: 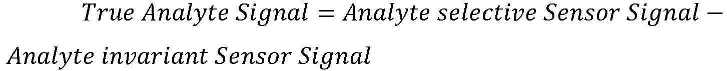 True Analyte Signal = Analyte selective Sensor Signal - Analyte invariant Sensor Signal

The above relation is implemented in a simple digital signal processing routine (such as a subtractor / difference engine) executed in device firmware or software. It can, likewise, be realized in simple analog hardware, such as a differential amplifier.

The common-mode signal that appears at both the analyte-selective and analyte-invariant sensors is extricated using a number of methods. Firstly, it is subtracted from the analyte signal by means of the subtractive relation: 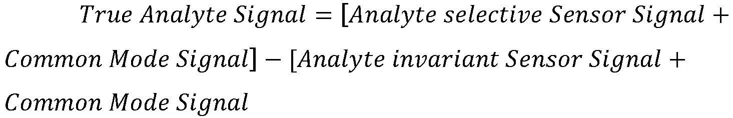 True Analyte Signal = [Analyte selective Sensor Signal + Common Mode Signal] - [Analyte invariant Sensor Signal + Common Mode Signal

The above is realized in a simple analog signal processing routine via a differential amplifier.

Assuming that the common-mode signal is not additive, but rather present in its entirety at the analyte-invariant sensor and as a modulation of the signal tendered by the analyte-selective sensor, the common-mode signal is ratiometrically extricated by the relation: $\begin{matrix}True Analyte Signal = \\ \frac{Analyte selective Sensor Signal ∗ Common Mode Signal}{Common Mode Signal}\end{matrix}$

Convolutional methods may be employed to extricate the pure analyte-selective signal component from other sources of noise. Assume the measured signal [m(x)] from the analyte-selective sensor represents the convolution of the component of the signal that is purely analyte-derived [a(x)] and a component imparted by sources of errant signal measures that are non-analyte in origin [n(x)], as measured by the analyte-invariant sensor:
$m \left(x\right) = a \left(x\right) ∗ n \left(x\right)$

Fourier- or wavelet-based decomposition of both the analyte-selective and analyte-invariant signals can be employed to spectrally discriminate between the analyte signal and the undue effect of any non-analyte-derived signal perturbations:
$m \left(x\right) = a \left(x\right) ∗ n \left(x\right) {\to }^{FT} M \left(jω\right) = A \left(jω\right) N \left(jω\right)$

In order to place equal weights on the spectral components, a normalization can be employed:
${M}_{NORM} \left(jω\right) = {A}_{NORM} \left(jω\right) {N}_{NORM} \left(jω\right)$

Or recasting:
${A}_{NORM} \left(jω\right) = {M}_{NORM} \left(jω\right) / {N}_{NORM} \left(jω\right)$

Hence the spectrally-pure tone arising from the analyte-selective signal is computed using the above relation. The inverse Fourier- or wavelet-transform is now employed to return to the time or data series domain:
${A}_{NORM} \left(jω\right) {\to }^{{FT}^{- 1}} {a}_{NORM} \left(x\right)$

The signal-to-noise ratio (SNR) engendered by such a system is computed as the logarithm (in base 10) of the ratio of analyte-selective sensor signal to the analyte-invariant sensor signal:
$SNR = 10 {log}_{10} \left(\frac{Analyte Selective Sensor Signal}{Analyte Invariant Sensor Signal}\right)$

This enables the calculation of the noise figure (NF) of the system:
$NF = \frac{{SNR}_{i}}{{SNR}_{o}}$

Where the SNRᵢ is the signal-to-noise ratio of the system at a specified analyte level and SNRₒ is the measured signal-to-noise ratio embodied by a particular measurement.

The common-mode rejection ratio (CMRR) is computed as the logarithm (in base 10) of the ratio of analyte-selective sensor signal to the analyte-invariant sensor signal:
$CMRR = 20 {log}_{10} \left(\frac{True Analyte Signal}{Analyte Invariant Sensor Signal}\right)$

Given the ability to measure the impact of non-analyte-derived signals, the following list of routines might be employed to compensate the non-analyte effects from the signal or produce an optimal estimate of the analyte concentration:

### Adaptive Filters:

In most signal processing applications⁵⁻⁷, the non-analyte effect is assumed to be additive, this is due to the fact that multiplicative models represent a greater challenge to solve. In these approaches, the general model at each discrete sample n is:
$s \left(n\right) = a \left(n\right) + i \left(n\right) + e \left(n\right)$

where *s(n)* is the total detected signal, *a(n)* is the desired analyte signal, *i(n)* is the additive contribution due to non-analyte contribution, and *e(n)* is the filter residual. In order to solve the equation above, an adaptive filter would adjust the coefficients of a time-varying filter *W(n)* to regress the non-analyte signal into *s(n).* The cost function is defined as:
$min \left\{norm\left(W′i-s,2\right)\right\} .$

At sample n, the filter residual is:
$e \left(n\right) = s \left(n\right) - {\sum }_{j = 1}^{p} {w}_{j} i \left(n-j\right) ,$
where p is the order of the filter. The residual is minimized to find the correlation between the reference interference signal and *s(n)* and the output may be classified as the 'cleaned' signal plus uncorrelated white noise. A wide variety of algorithms exists to solve this regression problem in real time such as: Recursive Least Squares (RLS); Least Mean Squares (LMS); Kalman Filter (KF); and Kernel Adaptive filtering (KAF).

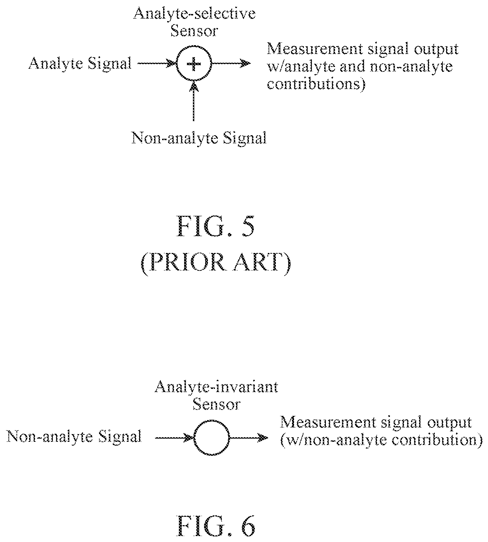

FIGS. 5-10 show block diagrams of the process flow of the sensors.

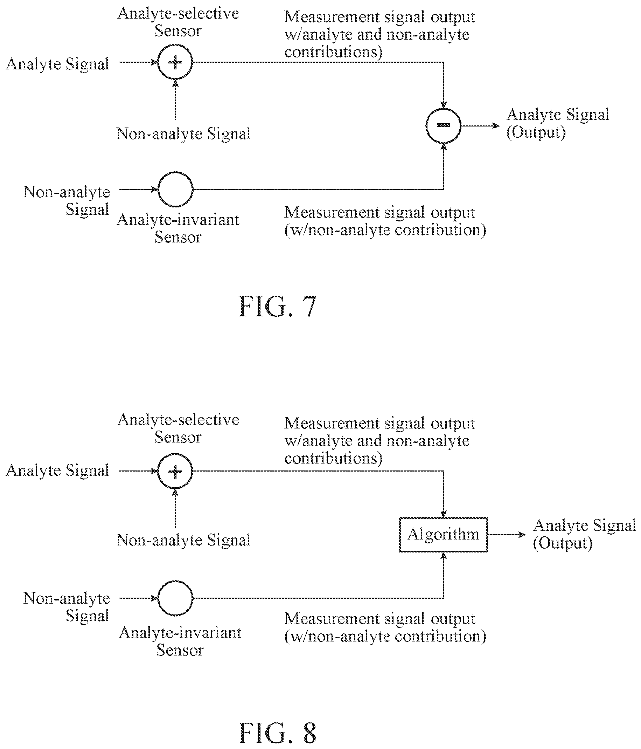

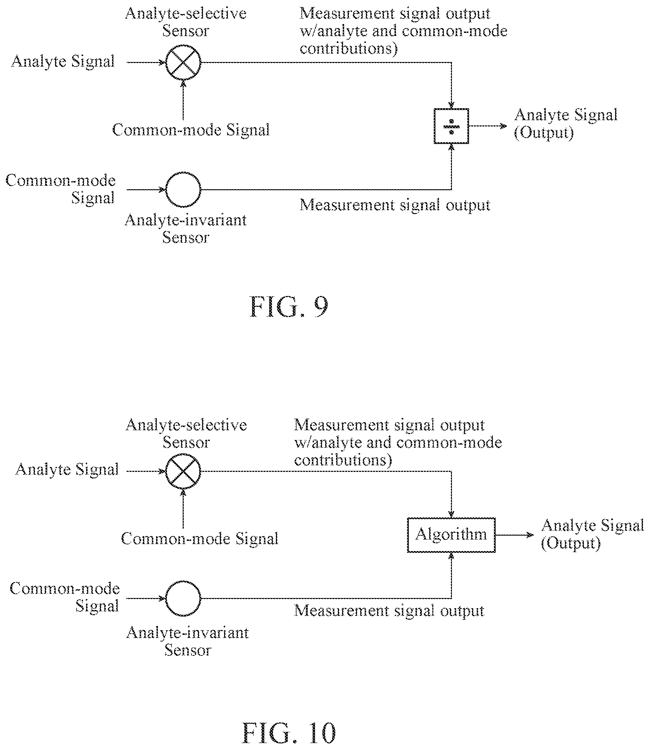

FIG. 5 shows a prior art analyte-selective sensor block diagram. The non-analyte signal is additive to the analyte signal. FIG. 6 shows an analyte-invariant sensor. FIG. 7 shows a system to remove the perturbations to the analyte signal that are non-analyte (and additive) in origin. FIG. 8 shows a system to remove the perturbations to the analyte signal that are non-analyte (and additive) in origin. FIG. 9 shows a system to remove the common-mode signal arising from perturbations that are non-analyte in origin. In this embodiment, the analyte signal is modulated by the common-mode signal whereas the analyte-invariant sensor is directly sensitive to the common-mode signal. FIG. 10 shows a system to remove the common-mode signal arising from perturbations that are non-analyte in origin. In this embodiment, the analyte signal is modulated by the common-mode signal whereas the analyte-invariant sensor is directly sensitive to the common-mode signal.

FIG. 11 shows a block diagram 180 of a device to remove signal perturbations that are non-analyte in origin. In certain embodiments, the analyte-selective sensor contains a membrane with an analyte / biorecognition element. In other embodiments, the analyte-invariant sensor contains a membrane lacking an analyte / biorecognition element. In yet other embodiments, the analyte-selective sensor and analyte-invariant sensor are at least two distinct electrodes. In other embodiments, the analyte-selective sensor is located on an electrode on at least one microneedle of a microneedle array. In yet other embodiments, the analyte-invariant sensor is located on at least one microneedle of a microneedle array. In another embodiment, the analyte sensor is a microneedle array. In other embodiments, the analyte sensor system is an analyte-selective microneedle array sensor. In other embodiments, the analyte-selective microneedle array sensor is body-worn on the skin surface of a user. In yet other embodiments, the algorithm is processed internally in the device. In other embodiments, the algorithm is processed in a wirelessly-connected device. In yet other embodiments, the algorithm is processed in a Cloud service. In yet other embodiments, the analyte measurement is provided to the user on a display. In other embodiments, the analyte measurement is used to guide therapeutic interventions in an automated insulin delivery system. In yet another embodiment, the analyte measurement is delivered to a wirelessly-connected device. In yet another embodiment, the analyte measurement is stored in a Cloud service.

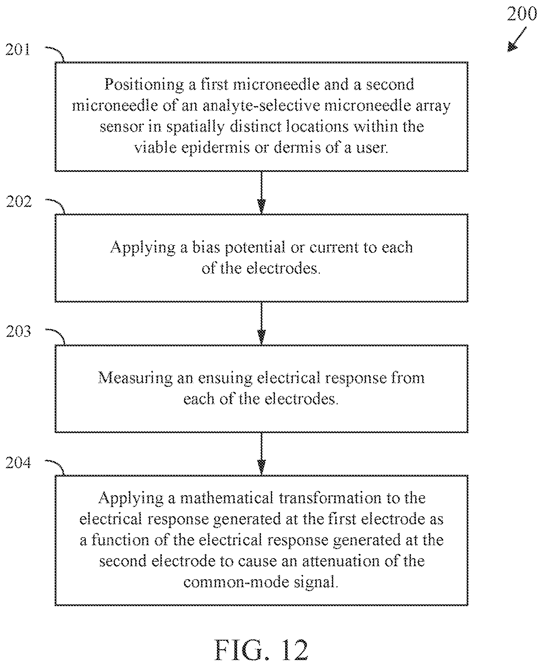

A method 200 for the mitigation of a non-analyte-derived signal perturbation incident upon a body-worn, microneedle array-based analyte sensor is shown in FIG. 12. Step 201 is positioning a first microneedle and a second microneedle of the microneedle array in spatially distinct locations within the viable epidermis or dermis of a user. Preferably, the first microneedle features a first electrode, a selective recognition element disposed on the first electrode and configured to generate a product arising from the interaction of the selective recognition element and the analyte, and a membrane disposed on the selective recognition element and the second microneedle features a second electrode and a membrane disposed on the second electrode. Step 202 is applying a bias potential or current to each of the first and second electrodes. Step 203 is measuring an ensuing electrical response from each of the first and second electrodes. Finally, step 204 is applying a mathematical transformation to the electrical response generated at the first electrode as a function of the electrical response generated at the second electrode to cause an attenuation of the common-mode signal.

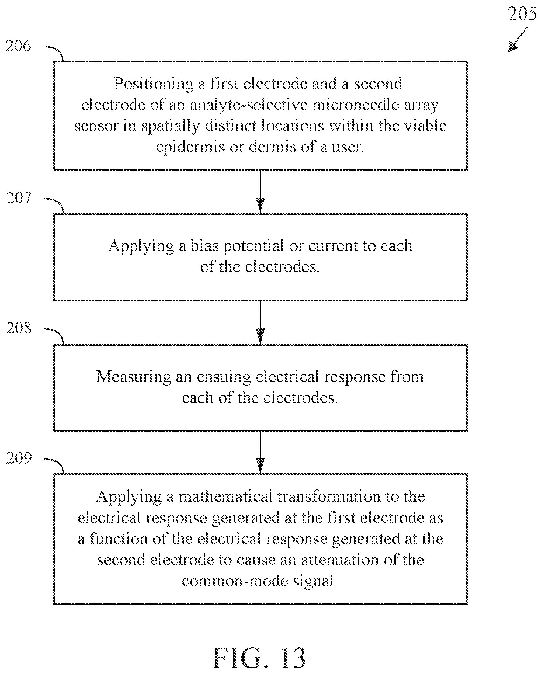

Another method 205 method for the mitigation of a non-analyte-derived signal perturbation incident upon a body-worn, analyte sensor is shown in FIG. 13. Step 206 starts with positioning a first electrode and a second electrode of the analyte sensor in spatially distinct locations within the viable epidermis or dermis of a user. Preferably, the first electrode features a selective recognition element disposed on the first electrode and configured to generate a product arising from the interaction of the selective recognition element and the analyte, and a membrane disposed on the selective recognition element and the second electrode features a membrane disposed on the second electrode. Step 207 is applying a bias potential or current to each of the first and second electrodes. Measuring an ensuing electrical response from each of the first and second electrodes is step 208. Step 209 is applying a mathematical transformation to the electrical response generated at the first electrode as a function of the electrical response generated at the second electrode to cause an attenuation of the common-mode signal.

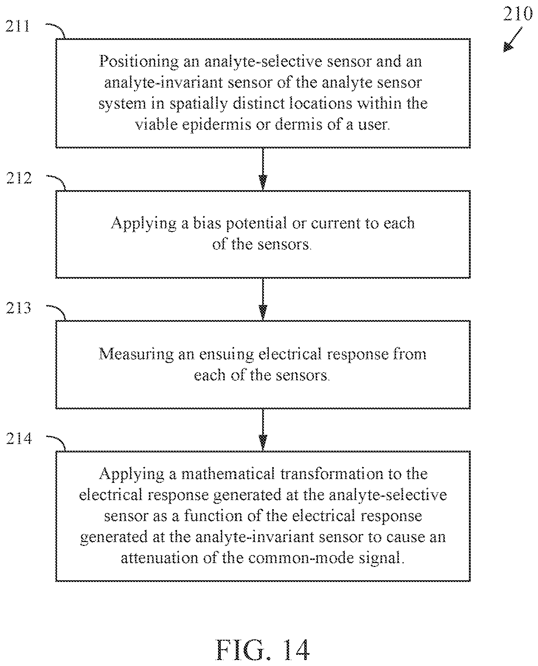

Yet another method 210 for the mitigation of a non-analyte-derived signal perturbation incident upon a body-worn, analyte sensor system is shown in FIG. 14. Step 211 is positioning an analyte-selective sensor and analyte-invariant sensor of the analyte sensor system in spatially distinct locations within the viable epidermis or dermis of a user. Preferably, the analyte-selective sensor features a first electrode, a selective recognition element disposed on the first electrode and configured to generate a product arising from the interaction of the selective recognition element and the analyte. Further, a membrane disposed on the selective recognition element, and the analyte-invariant sensor features a second electrode and a membrane disposed on the second electrode. Applying a bias potential or current to each of the analyte-selective sensor and analyte-invariant sensor is step 212. Step 213 is measuring an ensuing electrical response from each of the analyte-selective sensor and analyte-invariant sensor. Step 214 is applying a mathematical transformation to the electrical response generated at the analyte-selective sensor as a function of the electrical response generated at the analyte-invariant sensor to cause an attenuation of the common-mode signal.

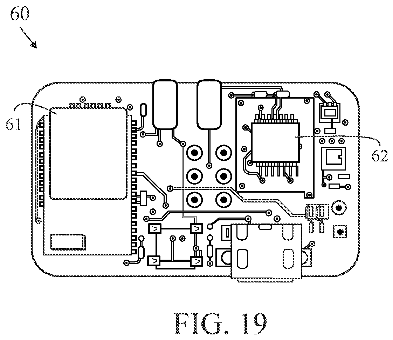

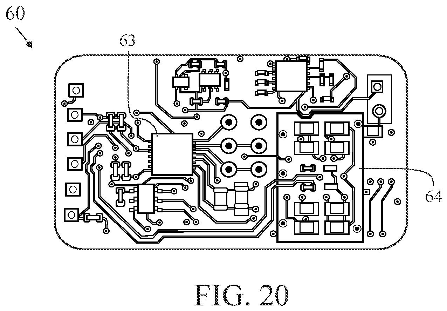

FIG. 19 illustrates the electronic circuitry contained in a wearable device enclosure 60 designed to interface directly with a microneedle-based biosensor device. The electronic circuitry of the device comprises a wireless transceiver (preferably BLUETOOTH LOW ENERGY) and a microcontroller with an integrated analog-to digital converter 61, and a high amplification circuit 62. FIG. 20 illustrates another view of the electronic circuitry contained in prototype wearable device enclosure 60 designed to interface directly with a microneedle-based biosensor device. The electronic circuitry comprises a high-sensitivity electrochemical analog front end 63 and a filtering circuit 64.

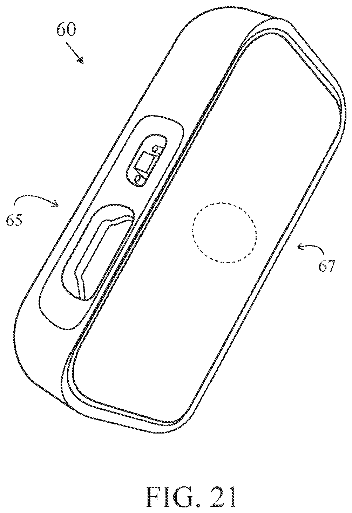

FIG. 21 illustrates the electronic circuitry contained in the wearable device enclosure 60 with access to the microneedle device provided via gold-plated pressure connectors 67 located on the viewable surface of the wearable device enclosure 60. A connection port 65 is also shown.

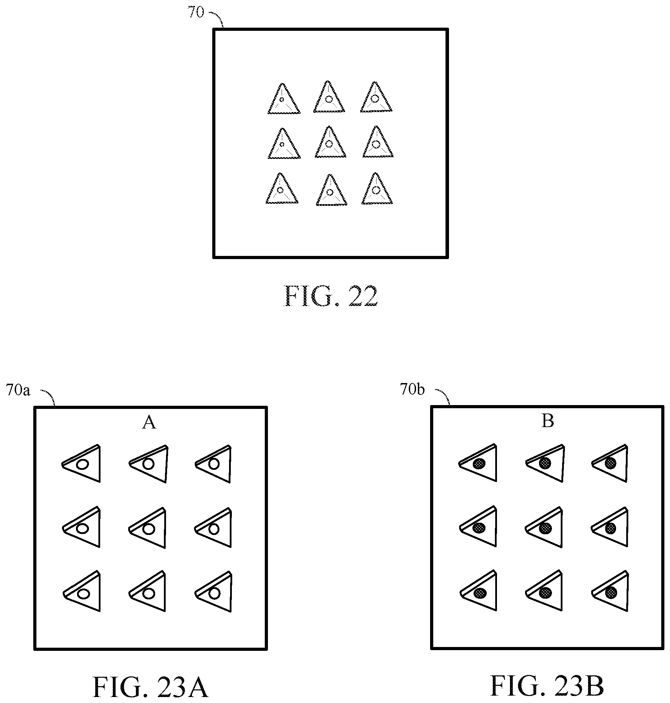

FIG. 22 illustrates a skin-penetrating hollow microneedle array 70 comprising a plurality of protrusions having vertical extent of approximately 1000 µm, with each element of the microneedle array functionalized to impart selective biosensing ability. FIG. 23A illustrates a hollow, unfunctionalized microneedle array 70a. FIG. 23B illustrates a hollow "filled", functionalized microneedle array 70b with selective biosensing ability.

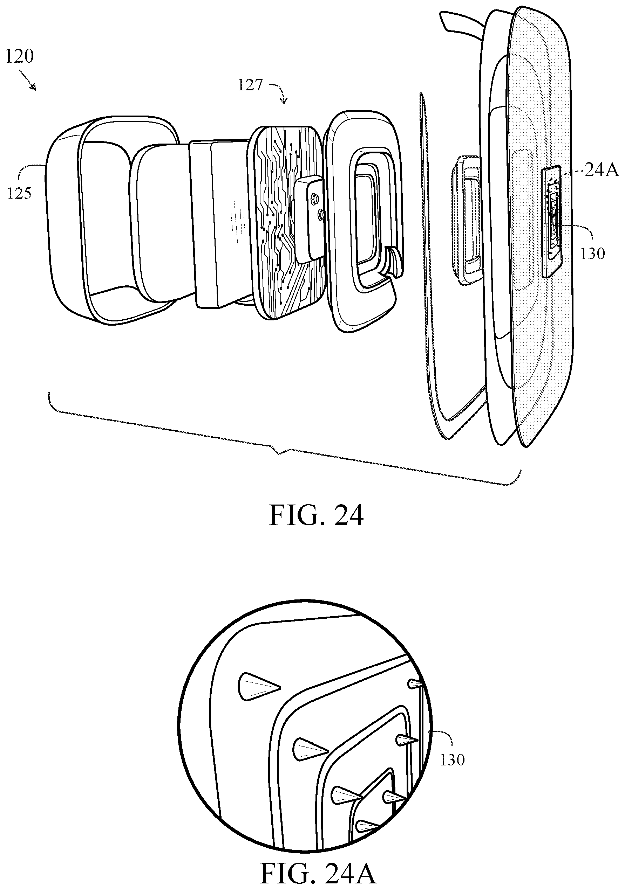

FIGS. 24 and 24A illustrate an exploded view rendering of complete microneedle biosensing system 120 illustrating the functional components, including a housing member 125, a microneedle biosensor 130 and a printed circuit board 127 containing the electronic circuitry required to transduce biochemical signals to digital data that are wirelessly transmitted to an external device via the embedded wireless transceiver.

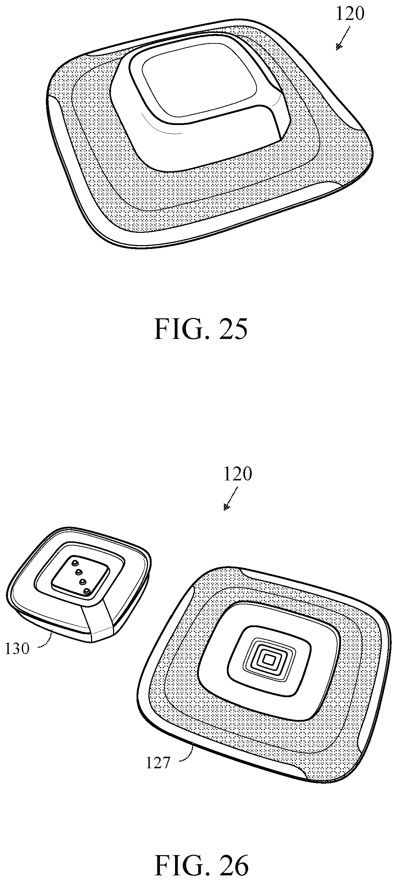

FIG. 25 illustrates a top perspective view of the wearable microneedle biosensing system 120 containing the electronic backbone (protrusion) and adhesive patch. The microneedle is located on the posterior surface of the adhesive patch (not shown).

FIG. 26 illustrates a posterior surface view of the electronics components housing constituent 130 of the microneedle-based biosensing system 120 and the skin-worn adhesive patch containing the microneedle array 127.

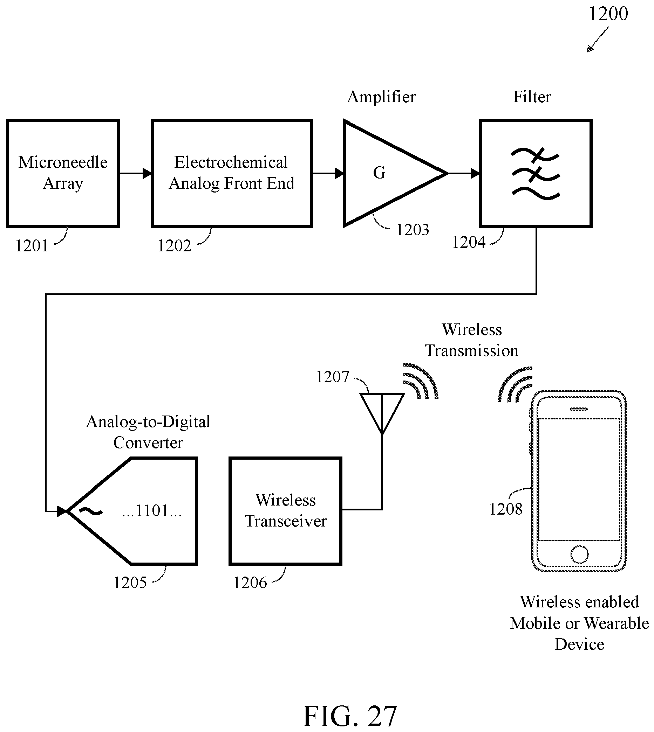

FIG. 27 illustrates a detailed block/process flow diagram 1200 illustrating the major functional components of the microneedle-based biosensing system and supporting electronic systems. At block 1201 is the microneedle array utilized to obtain transdermal biochemical analytes from a viable physiological medium (interstitial fluid, blood) occupying the layers of the epidermis and dermis of a user of the microneedle-based biosensing system. At block 1202, the electrochemical analog front end performs one (or more) of a number of electroanalytical techniques, such as voltammetry, amperometry, potentiometry, conductimetry, impedimetry, and polarography, to facilitate the control and readout of the electrochemical reaction occurring at the microneedle-based biosensing system. At block 1203, the electrical signal generated at the output of the electrochemical analog front end is directed to an amplification circuit to increase the signal strength to line levels. At block 1204, the output from the amplification circuit is directed to a low- or band-pass filter to extract a signal of interest and remove any undesired noise. At block 1205, the signal subsequently undergoes analog-to-digital conversion at an ADC to convert the analog signal to a digital bitstream. At block 1206, the signal is routed to a wireless transmitter or transceiver (BLUETOOTH, WiFi, RFID / NFC, Zigbee, Ant+) 1207 for transmission of the signal (corresponding to the level of the biochemical analyte) to a mobile communication device 1208 for further information processing, interpretation, display, archiving, and trending.

The electrochemical analog front end preferably includes: a Texas Instruments LMP91000 Sensor AFE System, configurable AFE potentiostat for low-power chemical sensing applications; a Texas Instruments LMP91200 configurable AFE for low-power chemical sensing applications; or an Analog Devices ADuCM350 16-Bit Precision, low power meter on a chip with Cortex-M3 and connectivity. The wireless transceiver is preferably is a BLUEGIGA BLE-113A BLUETOOTH Smart Module, or a Texas Instruments CC2540 SimpleLink BLUETOOTH Smart Wireless MCU with USB. The accompanying mobile device is preferably an ANDROID^{™}- or iOS^{™}-based smartphone, Samsung GALAXY GEAR, or an APPLE WATCH^{™}.

The microneedle array electrochemical biosensor transduces biochemical signals from the interstitial fluid into useful electrical signals.

The electrochemical analog front end preferably performs at least one or more of the following: applies a fixed potential or time-varying potential to the microneedle array to induce an electrochemical reaction, thereby giving rise to a flow of current; applies a fixed current or time-varying current to the microneedle array to induce an electrochemical reaction, thereby giving rise to an electrical potential; measures a time-varying open-circuit potential generated by an electrochemical reaction or ionic gradient; measures a frequency-dependent impedance generated by an electrochemical or bio-affinity reaction at the microneedle transducer; and measures a specific resistance or conductance generated by an electrochemical or bio-affinity reaction at the microneedle transducer.

The electrochemical analog front end is preferably dynamically configured to achieve any one of the above-numerated embodiments. Likewise, the inputs are preferably arrayed to operate sequentially or in parallel to expand the sensing capabilities of the system.

The wireless transceiver wirelessly relays electrical signals generated by the electrochemical analog front end to a mobile or wearable device using any one of a number of standardized wireless transmission protocols (Bluetooth, WiFi, NFC, RFID, Zigbee, Ant+). Optionally, the electrical signal generated by the analog front end can be amplified, filtered, and/or undergo analog-to-digital-conversion and further signal processing prior to being relayed by the wireless transceiver.

The mobile or wearable device displays sensor readings to the user in an easily-understood format, and performs any additional signal processing necessary.

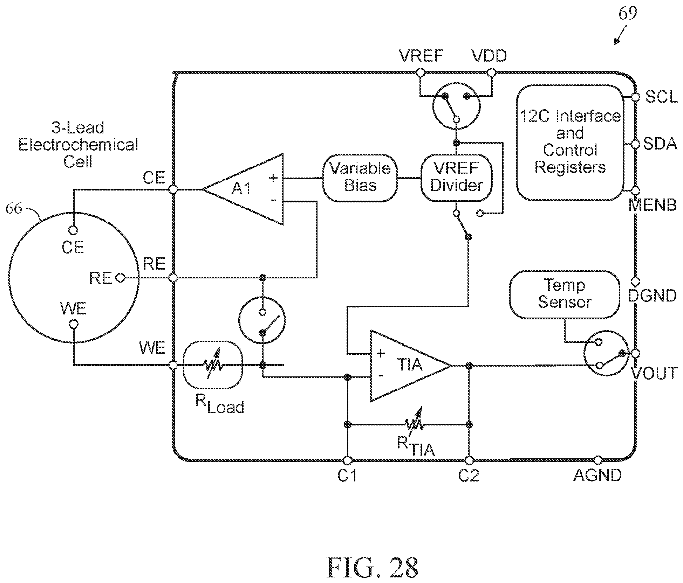

As shown in FIG. 28, an adjustable bias analog front end / potentiostat 69 is composed of high-input impedance operational amplifiers and a digital to analog converter, or a standalone analog front end ("AFE") or analog interface integrated circuit package.

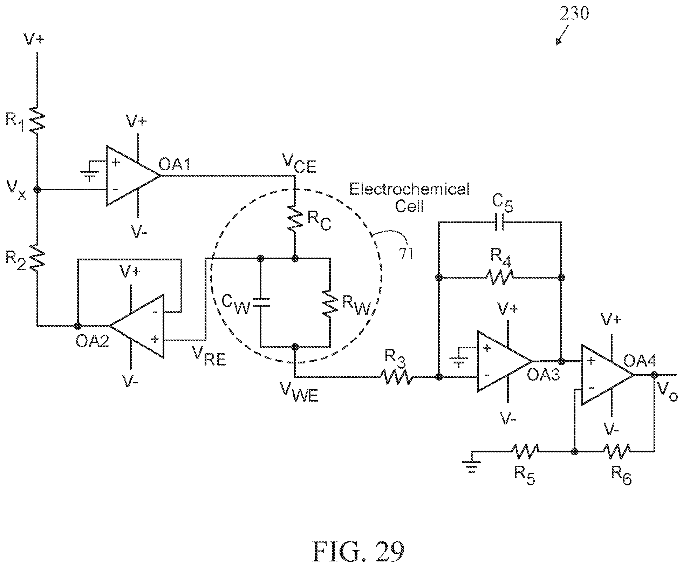

FIG. 29 is a circuit diagram of a multi-component potentiostat 230 with an electrochemical cell 71.

The method steps of the potentiostat operation are as follows:
The Analog Front End/Potentiostat Operation. The potentiostat/AFE unit consists of either two (FIG. 28) or three (FIG. 29) precision instrumentation operational amplifiers (A1/OA1, OA2, and TIA/OA3) configured in the following arrangement: control amplifier A1/OA1 amplifies the differential voltage (Vₓ in FIG. 20) measured between a variable (programmable) bias and ground (with gain A) and supplies current through the counter electrode (CE). Upon sensing a voltage generated at the reference electrode (RE), A1/OA1 sinks sufficient current in order to maintain its output voltage at the input (V_{RE}) value. In turn, RE is adjusted and the output potential/current of A1/OA2 (a buffer or unity-gain amplifier) is modified accordingly. The control amplifier thus functions as a voltage-controlled current source that delivers sufficient current to maintain the reference electrode at constant potential and arbitrate the electrochemical reaction. In implementing negative feedback, it is imperative that A1/OA2 be able to swing to extreme potentials to allow full voltage compliance required for chemical synthesis. Furthermore, it is crucial that the OA2 possesses very high input impedance in order to draw negligible current; otherwise the reference electrode may deviate from its intended operating potential. In practice, the use of precision amplifiers possessing 20 fA (or lower) of input bias current enables unabated operation to the sub-picoampere level, which is suitable for nearly all electrochemical studies. The TIA/OA3 accepts the current sourced through the working electrode (WE) and outputs a voltage (converted by resistor/capacitor network R_{TIA}/C₅+R₄) proportional to the amount of current passing through electrode WE.

The Analog Front End and Applied Reference/Working Bias. In the system shown in FIGS. 28 and 29, the reference voltage (V_{RE} / RE) is held constant at the inverting and noninverting inputs for operational amplifier A1/OA2, respectively, while the working voltage is changed through a voltage divider, resistor network, or other means, to create an operational bias on the connected sensor. Current passing from CE to WE is directed into the noninverting input of a variable-gain transimpedance amplifier, which converts the current flow into a scaled voltage output (at C2 and/or VOUT/Vo) according to the relation VOUT/Vo = -i_{cell}R_{4/TIA}.

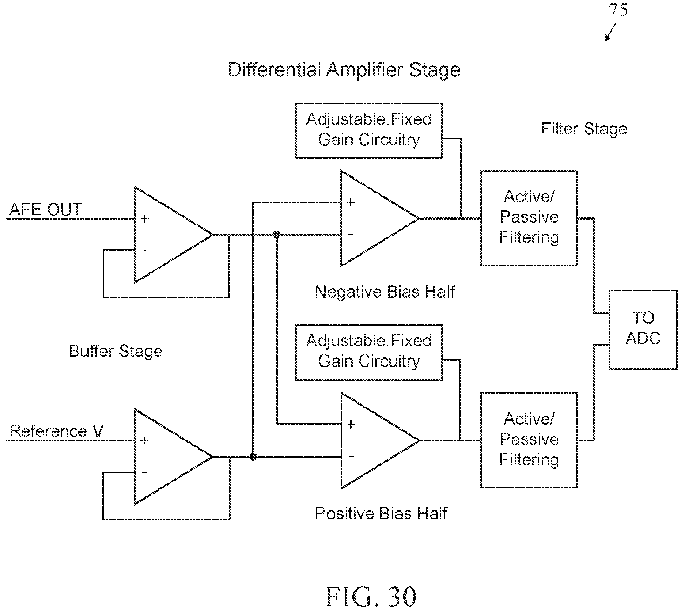

The difference amplifier stage 75 is shown in FIG. 30. The difference amplifiers are configured to accept the applied reference voltage (RE or C1 in the internal IC diagram) and the output from the transimpedance amplifier (with or without a buffer stage). The inputs are juxtaposed among the two amplifiers, namely the reference input is connected to the positive terminal on one of the amplifiers (for negative applied voltages/currents) and on the negative terminal of the other (for positive applied voltages/currents). VOUT is connected to the opposing amplifier input. The unused amplifier (opposing the polarity of the applied current/voltage) will have its inputs driven to zero; it will, however, still possess a ground bias if one is present within the system. The gain of the difference amplifier can be configured either through manufacture or in real time to scale to the amount of voltage/current read in by the AFE.

The Filtering step. The outputs generated from the difference amplifier pair are subsequently subjected to a filtering circuit to remove extraneous noise. Oscillations or random fluctuations in the signal can be present due to a number of reasons, including ground bias, RF interference, mains power oscillation, input impedance mismatch (from the 3 electrode sensor), or from other sources.

The Analog to Digital Converter step. The filtered signals are lastly incident upon an analog to digital converter ("ADC"), either located in an external integrated circuit ("IC"), or co-located within a microcontroller or other IC, and converted into a representative digital signal. Increased sampling resolution may be implemented to gain additional sensitivity and minimize quantization error.

The Collection Algorithm step. To further reduce noise, a time averaged value for both positive and negative bias lines will be collected and computed by a microcontroller/microprocessor over a period of a few seconds (subsequent to digitization by the ADC). The active bias amplifier (applied voltage/current) will have the value of the inactive bias amplifier (ground offset) subtracted in order to remove any present bias in the device. Due to this process, a shielding cage is not required to reach picoampere levels of sensitivity. The inactive bias amplifier, time average data collection, and filtering schemes will provide a stable and scalable output into the microcontroller/processor at all times.

The input of the electrochemical cell or sensor, the analyte, is measured by controlled-potential techniques (amperometry, voltammetry, etc). The output of the sensing system, consisting of a measured voltage and calculated current value (determination of current flowing through working and counter electrodes of electrochemical cell or sensor), corresponds to the concentration of the analyte in the sample.

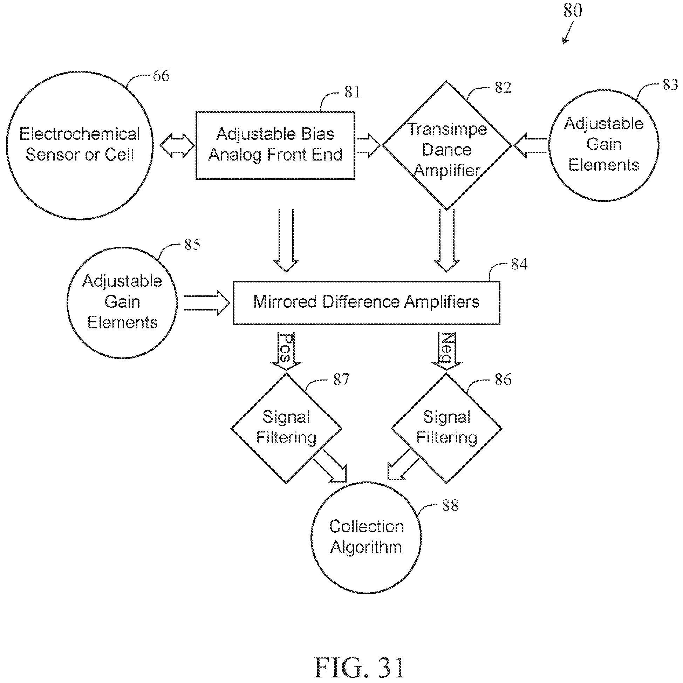

FIG. 31 illustrates a signal flow diagram 80 for detecting a current flowing an electrochemical cell. A current signal from an electrochemical cell 66 is sent to an adjustable bias analog front end 81. The signal is sent to a transimpedance amplifier 82. The signal is sent from both the adjustable bias analog front end 81 and the transimpedance amplifier 82 to mirrored difference amplifiers 84. The outputs generated from the mirrored difference amplifiers 84 are subsequently subjected to filtering circuits 86 and 87 to remove extraneous noise. Oscillations or random fluctuations in the signal can be present due to a number of reasons, including ground bias, RF interference, mains power oscillation, input impedance mismatch (from the 3 electrode sensor), or from other sources. At the collection algorithm 88, to further reduce noise, a time averaged value for both positive and negative bias lines is collected and computed by a microcontroller/microprocessor over a suitable time period, such as a few seconds (subsequent to digitization by the ADC). The active bias amplifier (applied voltage/current) will have the value of the inactive bias amplifier (ground offset) subtracted in order to remove any present bias in the device. Due to this process, a shielding cage is not required to reach picoampere levels of sensitivity. The inactive bias amplifier, time average data collection, and filtering schemes will provide a stable and scalable output into the microcontroller/processor/ADC at all times.

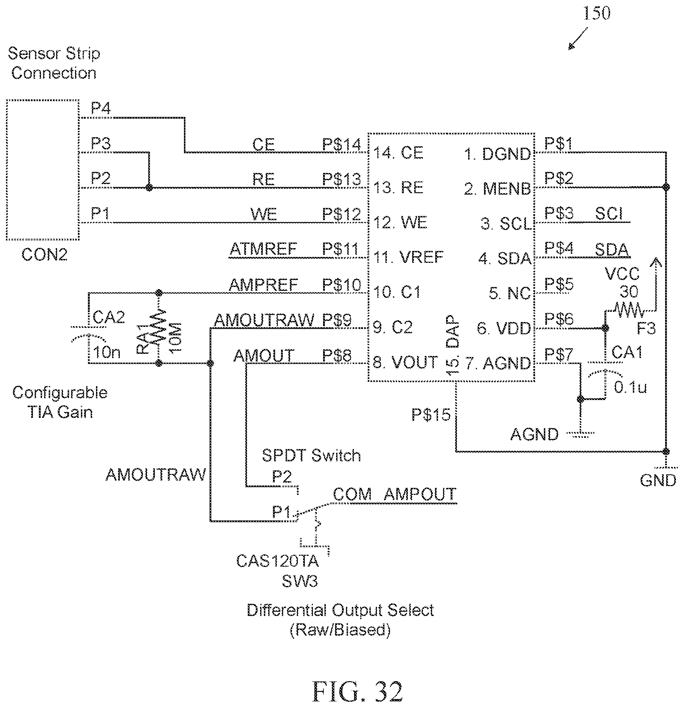

FIG. 32 is a detailed circuit diagram of an integrated analog front end 150 and sensor interface. This is a circuit diagram of an integrated AFE available from a manufacturer that communicates (SCL and SDA lines) with a central microcontroller/microprocessor unit and controls an electrochemical sensor via the CE (counter electrode), WE (working electrode), and RE (reference electrode) lines. The configurable circuit components for the transimpedance amplifier (TIA) are present across 9 and 10 and forms an integrator as configured in the image.

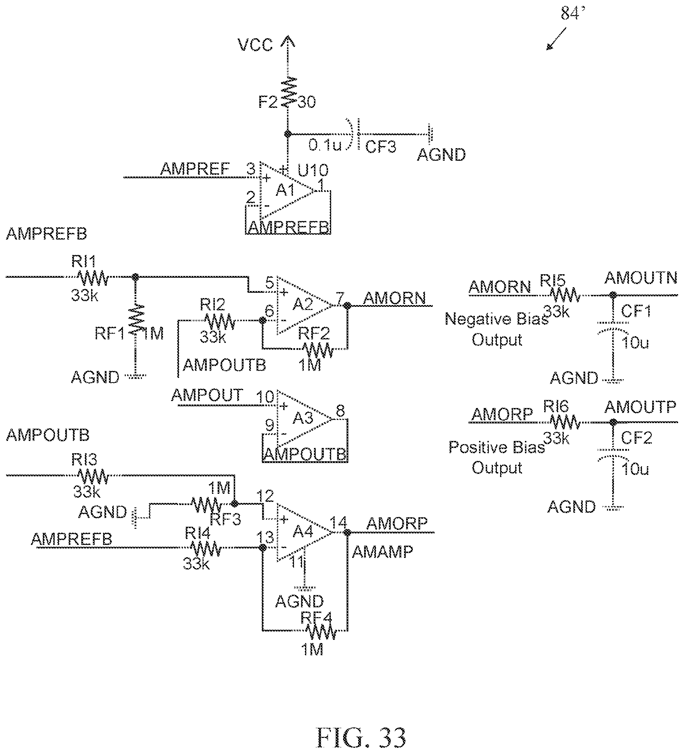

FIG. 33 is a detailed circuit diagram of mirrored difference amplifiers 84' and filtering. Here, a set of mirrored difference amplifiers is shown utilizing individual operational amplifier components (left side) and a low pass filter on the output(right side). AMORP and AMORN are the positive and negative differential signals, and AMOUTN and AMOUTP are the filtered differential signals. Output gain is controlled by the passive resistors connected to the amplifiers.

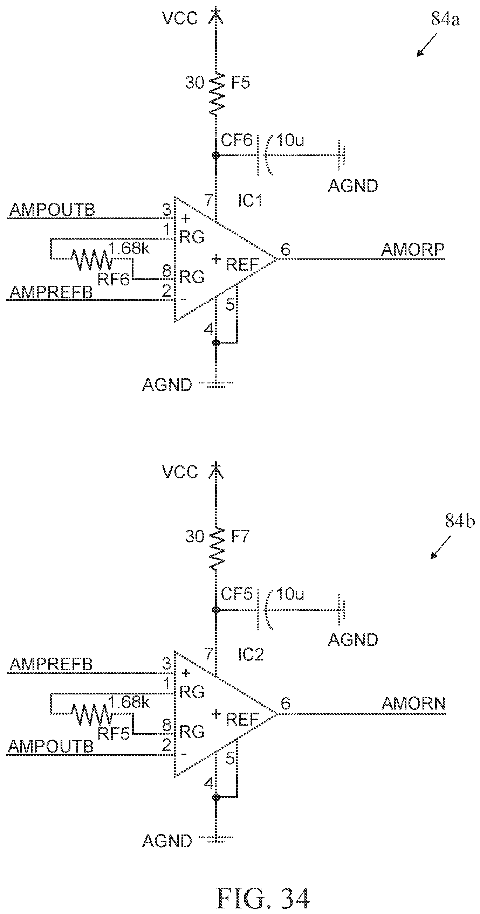

FIG. 34 is a detailed circuit diagram of fixed mirrored instrumentation amplifiers 84a and 84b. Here, a set of mirrored difference amplifiers is shown using a pair of integrated instrumentation amplifiers. Output gain is controlled by a single resistor connected to the RG terminals.

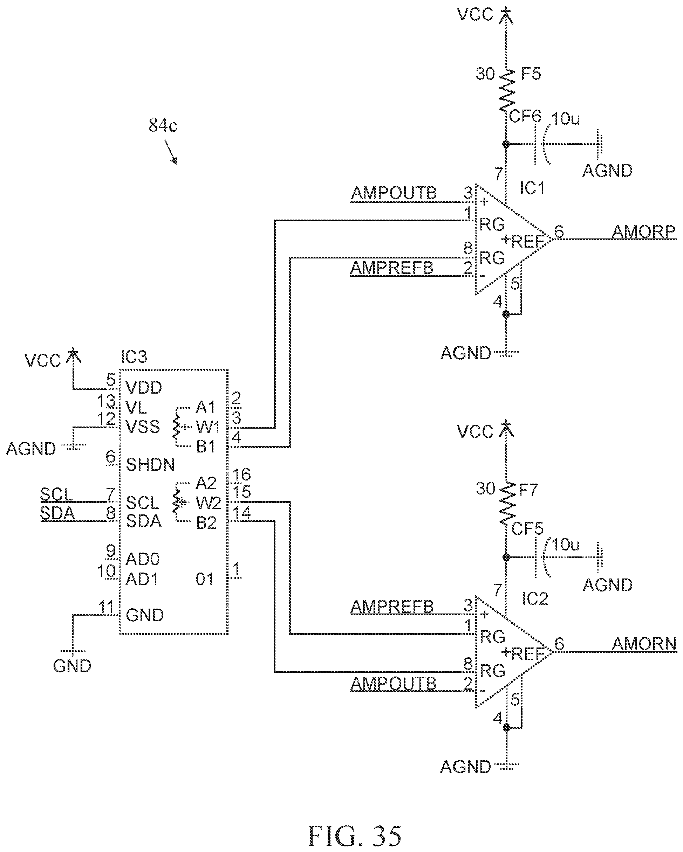

FIG. 35 is a detailed circuit diagram of digital potentiometer-adjustable mirrored instrumentation amplifiers 84c. This is similar to FIG. 34, albeit utilizing a programmable/digitally selectable gain resistor integrated circuit (IC3) rather than passive components.

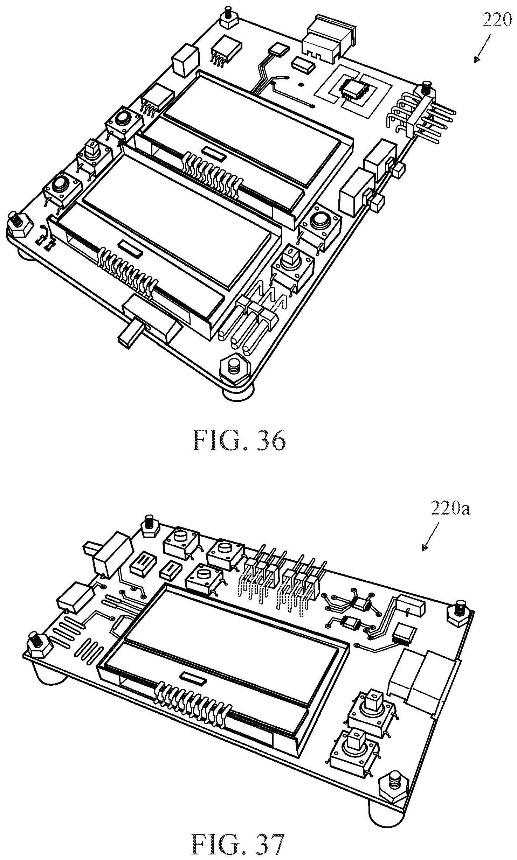

FIG. 36 is an illustration of a handheld analyzer 220 in a large form factor.

FIG. 37 is an illustration of a handheld analyzer 220a in a small form factor.

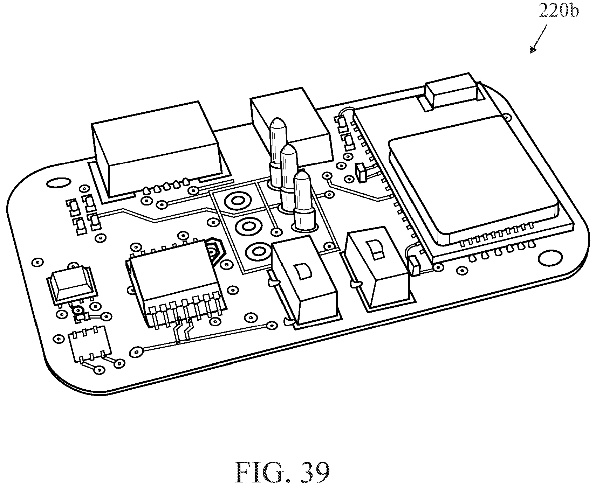

FIG. 39 is an illustration of a handheld analyzer 220b in a small form factor.

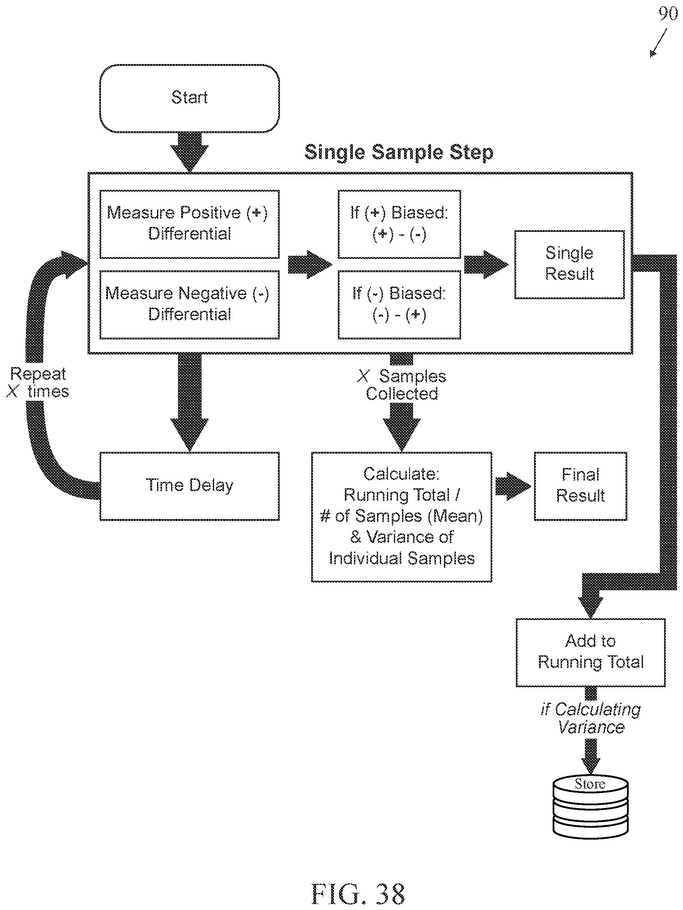

The sampling and measurement algorithm is designed to minimize sources of noise that are not compensated or otherwise removed using the circuit hardware. As shown in the block diagram 90 of FIG. 38, each "sample" involves reading both the positive and negative differential outputs and subtracting one from the other. Multiple samples can be collected and analyzed via statistical operations to yield a measurement. The simplest form is to calculate mean and variance/standard deviation from a set of individual samples. The sampling period has to be selected in a manner that minimizes the possibility of noise from other electrical sources.

The main sources of noise are: floating ground and ground drift; mains power; and high frequency interference.

The floating ground and ground drift are compensated by various means. Floating ground (DC noise) is compensated by the presence of the paired difference amplifiers. Ground drift is compensated by averaging multiple samples. If measuring a positive bias/current, the negative output will be equal to the floating ground. Subtracting the negative output from the positive will remove noise caused by ground drift. The opposite can be performed when measuring a negative bias/current. The subtraction step should be performed at each sample rather than using averages of multiple readings.

Mains Power is also compensated in various ways. Noise arising due to mains power when either connected to an AC power line or induced by proximity to other AC line-powered equipment is compensated by selection of the algorithm sampling period. Sampling should never be performed at the same delay as the period of the line power cycle (16 or 20 ms for 60 Hz and 50 Hz power systems, respectively) or any multiple thereof (i.e. 32 to 40 ms for a multiple of two, etc). If sampling delay is less than the line power cycle (16-20 ms), at least one cycle (at 50-60 Hz) must be captured by multiple samples. For proper statistical analysis, enough samples must be collected to establish an adequate estimate of the standard deviation and mitigate power line harmonics. For a 95% confidence interval for Type 1 (false positive) and Type 2 (false negative) errors, for example, at least 13 samples must be measured. This is application-specific but a minimum of 10 samples is recommended. The maximum sample number is application-dependent (the likelihood of sudden changes due to external factors, such as movement in the case of a body worn sensor).

High frequency interference, noise due to wireless transmission and other high frequency signals, is eliminated fully by hardware filtering, notably low pass filtering.

### Neural Networks:

A wide variety of neural networks (NNs) may be used to both fuse multichannel signal measurements and also remove the undesired signals. The input to the NN comprises the input measurements and the network is trained *a priori* on desired signal measurement (i.e. interstitial glucose values). The network is trained, using either supervised or unsupervised learning methods, to develop a mathematical model mapping between signal (i.e. electrical current), temperature, non-analyte and other sources of interference and the target desired analyte signal. Different forms of deep and shallow neural networks might be built with combination of following layers: Recurrent Neural Networks; Convolutional Neural Networks.

### Convex Optimization:

In a number of embodiments, real-time convex optimization is employed to deconvolve the undesired effects by constructing regression cost functions that have additional penalizing factors in their cost function in order to apply prior knowledge of smoothness or other frequency-based knowledge of the interreference signals.

### Projection Techniques:

Projection techniques such as linear and nonlinear (kernel) Principal Component Analysis (PCA) and Independent Component Analysis (ICA) are also employed in selected embodiments for blind source separation. In this case, the input matrix X contains all the signals, including analyte-selective and non-analyte-selective signals, as well as any extraneous signal readouts, such as temperature. These approaches create a rotation matrix A that maximizes the variance (in case of PCA) and independence (in case of ICA) that results in separation of the input sources.

### Continuous Wavelet Transform:

Continuous Wavelet Transform (CWT) of the analyte-selective and analyte-invariant sensor measurements are computed in certain embodiments so that a two-dimensional corresponding time-frequency of non-analyte and 'contaminated' analyte signal measurements can be constructed. The corresponding coefficients of frequencies that are correlated between the reference non-analyte and contaminated analyte signals in time are set to zero to remove the said effects.

Non-analyte-derived signal perturbations observable in analyte-selective sensors can claim origin from a plethora of physio-chemical processes, some of which are endogenous to the biological milieu while others arise due to exogenous effects instigated by the wearer of said sensors. Indeed, body-worn analyte-selective sensors often succumb to pressure-induced signal irregularities due to the inadvertent application of pressure or force onto the said sensor enclosure or housing; these are referred to as pressure-induced sensor attenuations (PISAs). This oftentimes is caused by induced changes in perfusion to the sensor or localized depletion of the analyte of interest or co-factor, such as oxygen. The disruption of the diffusion layer (nanometers to millimeters in extent) is also a cause of said PISA events since the sensing operation enabled by said analyte-selective sensors is diffusion-limited in nature. The execution of an analyte-invariant measure enables the identification of these instances, especially in the acute phase, as it is generally understood that the response of said analyte-invariant sensor is largely immune to said PISA events. Moreover, all electrochemical sensors undergo a non-Faradaic process immediately following excitation with an electrical stimulus wherein the ensuing signal response is not proportional to analyte concentration by the Cottrell relation, but rather the charging of the double-layer capacitance through the solution resistance. This is always manifested upon excitation of an electrochemical sensor with a voltage or current stimulus and decays to negligible levels in a finite time according to the *RₛC_{dl}* time constant, where *Rₛ* is the solution resistance and *C_{dl}* is the double layer capacitance. An analyte-invariant sensor undergoing the same non-Faradaic signal decay as an analyte-selective sensor may be employed in a differential configuration to extricate the true analyte signal from the non-Faradaic signal response. Similarly, implanted analyte-selective sensors require a certain time duration prior to measurement of accurate representations of analyte levels, which referred to as 'warm-up time' or 'burn-in'. The said warm-up or burn-in process is a complex physio-chemical interaction, which is governed by an interplay between hydration of the sensor membrane(s), establishment of equilibrium between the sensor membrane(s) and the surrounding interstitial medium, and adsorption of the circulating endogenous proteins (occupying the interstitial space) on the sensing surface of said analyte-selective sensor. An analyte-invariant sensor undergoing the same warm-up process as an analyte-selective sensor may be employed in a differential configuration to extricate the true analyte signal from the non-Faradaic signal response and hence yield measurements in a more timely fashion following sensor application, as shown in FIGS. 3A-3C and FIG. 4.

The preferred embodiments of the current disclosure include the removal of said non-analyte signal perturbation(s) in the system's analog front end, sensor front end, embedded computer, microprocessor, microcontroller, in a wirelessly-connected mobile device such as a smartphone, smartwatch, or tablet, or in a Cloud service. In other embodiments, the geometry and/or constituency of the analyte-invariant sensor is identical to that of the analyte-selective sensor with the exception that the biorecognition element (i.e. enzyme, antibody, aptamer) is absent. In yet other embodiments, the geometry and/or constituency of the analyte-invariant sensor is identical to that of the analyte-selective sensor with the exception that the biorecognition element (i.e. enzyme, antibody, aptamer) is inactive or has been rendered inactive during the manufacturing process. In yet other embodiments, the system contains a plurality of analyte-selective sensors and a single analyte-invariant sensor. In yet other embodiments, the system contains a plurality of analyte-selective sensors, each selective towards a *unique* analyte, and at least one analyte-invariant sensor. In yet other embodiments, readout from the analyte-invariant sensor is utilized to extricate and remove a temperature dependency of the analyte-selective sensor. In yet other embodiments, readout from the analyte-invariant sensor is utilized to extricate and remove interference from co-circulating analytes to which the analyte-selective sensor might exhibit partial sensitivity. In yet other embodiments, the current method of mitigation of non-analyte signal perturbations incident upon analyte-selective sensors is employed in a sensor fusion algorithm to improve reliability and/or accuracy of the measure of the analyte(s) of interest. In yet other embodiments, the analyte-selective and analyte-invariant sensors occupy the same microneedle constituent within a microneedle array.

An ARRAY is a microneedle or microneedle array-based electrochemical, electrooptical, or fully electronic device configured to measure an endogenous or exogenous biochemical agent, metabolite, drug, pharmacologic, biological, or medicament in the dermal interstitium, indicative of a particular physiological or metabolic state in a physiological fluid of a user. Specifically, said microneedle array contains a plurality of microneedles, possessing vertical extent between 200 and 2000 µm, configured to selectively quantify the levels of at least one analyte located within the viable epidermis or dermis and in the vicinity of the papillary plexus, subpapillary plexus, or dermal plexus. Said microneedle array is contained and/or mounted to an enclosure or housing containing a power source, electronic measurement circuitry, a microprocessor, and a wireless transmitter. Sensor is configured with a skin-facing adhesive (sensor adhesive) intended to adhere the said sensor for the desired wear duration.

Analyte-selective sensor (SELECTIVE SENSOR) is an electrode on the surface of at least one microneedle of said microneedle array, a selective recognition element disposed on said electrode and configured to generate a product arising from the interaction of said selective recognition element and an analyte indicative of a particular physiological or metabolic state in a physiological fluid of a user, and a membrane disposed on said selective recognition element. Said analyte is comprised of at least one endogenous or exogenous biochemical agent, metabolite, drug, pharmacologic, biological, or medicament.

Analyte-invariant sensor (INVARIANT SENSOR) is an electrode on the surface of at least one microneedle of said microneedle array distinct from SELECTIVE SENSOR and a membrane disposed on said electrode.

An Algortihm (ALGORITHM) is a mathematical transformation applied to the electrical response generated at the SELECTIVE SENSOR as a function of the electrical response generated at the INVARIANT SENSOR to remove the common-mode signal present at both said sensors.

In a method, a measurement is recorded at SELECTIVE SENSOR. A qualitative or quantitative determination of the level of a target biomarker, chemical, biochemical, metabolite, electrolyte, ion, hormone, neurotransmitter, vitamin, mineral, drug, therapeutic, toxin, enzyme, protein, nucleic acid, DNA, or RNA circulating within a physiological fluid of a user. Next, an ALGORITHM is applied to the measurements recorded at SELECTIVE SENSOR and INVARIANT SENSOR. A mathematical transformation is thus applied to the electrical response generated at the SELECTIVE SENSOR as a function of the electrical response generated at the INVARIANT SENSOR to remove the common-mode signal present at both said sensors. Said algorithm can comprise of at least one of a difference operation, denoising operation, regression, deconvolution, Fourier decomposition, background subtraction, Kalman filtering, and Maximum Likelihood Estimation.

Inputs of the disclosure include an analyte measurement and an analyte-invariant measurement. The analyte measurement is a qualitative or quantitative determination of the level of a target biomarker, chemical, biochemical, metabolite, electrolyte, ion, hormone, neurotransmitter, vitamin, mineral, drug, therapeutic, toxin, enzyme, protein, nucleic acid, DNA, or RNA circulating within a physiological fluid of a user. Measurement is provided by SELECTIVE SENSOR. The analyte-invariant measurement is a qualitative or quantitative determination of any endogenous or exogenous, stochastic or non-stochastic, physical and/or chemical processes incident upon analyte-selective electrochemical sensors that are non-analyte-related in origin. These processes often serve to corrupt the measurement signal tendered by said analyte-selective sensors. Measurement is provided by an INVARIANT SENSOR.

The output of the disclosure is an analyte measurement with common mode signal removed, which is a qualitative or quantitative measurement of the endogenous levels of a particular analyte of interest.

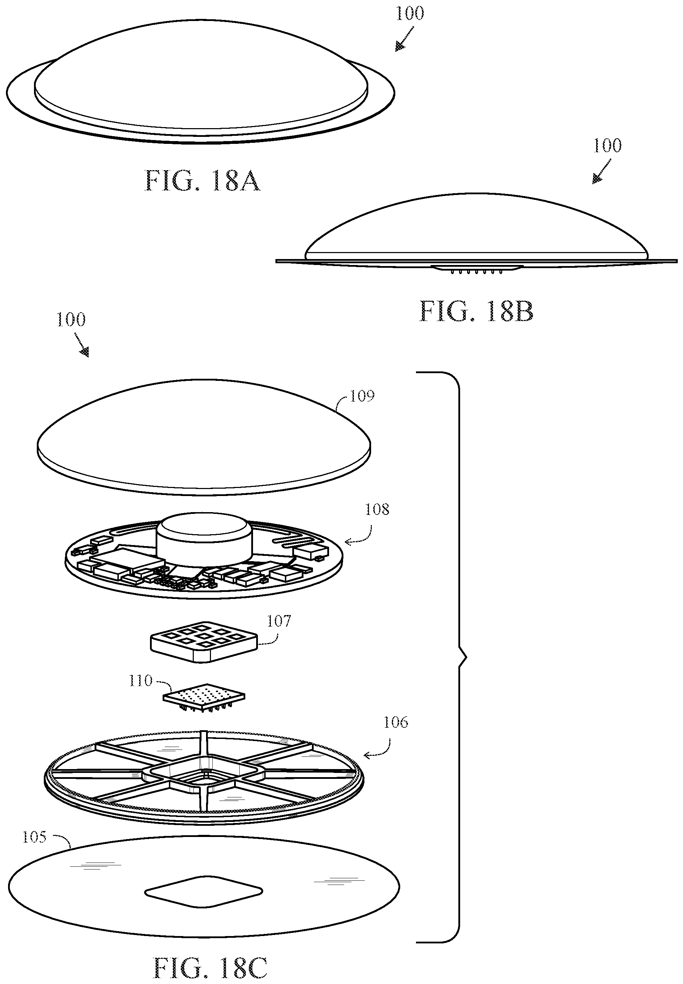

FIGS. 18A-18C are illustrations of a sensor. FIG. 18C is an exploded rendering of a microneedle sensor 100 illustrating the main components, including a cover 109, a main board with battery 108, a connector board 107, a microneedle array 110, a base with seals 106, and an adhesive patch 105.

## Claims

1. A device comprising:
a microneedle array (70) comprising:
a first microneedle (30a) comprising a first analyte-selective electrode;
a second microneedle (30b) comprising a second analyte-invariant electrode (40b); and
a selective recognition (41) element disposed on the first electrode and configured to generate a first electrical response arising from an interaction of the selective recognition element and an analyte; and
a processor,
wherein the first microneedle and the second microneedle are configured to be positioned within a viable epidermis or a dermis of a user,
wherein the processor is configured to measure the first electrical response comprising a first impedance from the first electrode and a second electrical response comprising a second impedance from the second electrode, the first electrical response and the second electrical response resulting from application of a bias potential applied to each of the first electrode and the second electrode, and
wherein the processor is configured to determine a resultant signal representative of the first electrical response generated at the first electrode and the second electrical response generated at the second electrode based on a ratio of the first electrical response and the second electrical response.

2. The device of claim 1, wherein the first electrode further comprises a first membrane disposed on the selective recognition element.

3. The device of claim 2, wherein the second electrode comprises a second membrane disposed on the second electrode.

4. The device of claim 3, wherein the first membrane and the second membrane comprise the same material.

5. The device of claim 1, wherein the first impedance comprises a first frequency-dependent impedance and the second impedance comprises a second frequency-dependent impedance.

6. The device of claim 1, wherein the bias potential applied to the first electrode and to the second electrode is identical.

7. The device of claim 1, wherein the bias potential applied to the first electrode and to the second electrode comprises an AC potential.

8. The device of claim 1, wherein the resultant signal comprises an analyte signal representative of a concentration of the analyte at the first electrode.

9. The device of claim 1, wherein each of the first electrode and the second electrode is disposed at a distal end of the respective microneedle.

10. A method comprising:
providing a first microneedle (30a) comprising a first electrode (40a) and a second microneedle (30b) comprising a second electrode (40b) each configured to be positioned within a viable epidermis or a dermis of a user, wherein a selective recognition element (41) is disposed on the first electrode and configured to generate a first electrical response arising from an interaction of the selective recognition element and an analyte;
applying a bias potential to each of the first electrode and the second electrode;
measuring the first electrical response from the first electrode and a second electrical response from the second electrode, wherein the first electrical response comprises a first impedance from the first electrode and the second electrical response comprises a second impedance from the second electrode; and
determining a resultant signal representative of the first electrical response generated at the first electrode and the second electrical response generated at the second electrode based on a ratio of the first electrical response and the second electrical response.

11. The method of claim 10, wherein the first electrode further comprises a first membrane disposed on the selective recognition element.

12. The method of claim 10, wherein the second electrode comprises a second membrane disposed on the second electrode, and wherein the first membrane and the second membrane comprise the same material.

13. The method of claim 10, wherein the bias potential applied to the first electrode and to the second electrode is identical or the bias potential applied to the first electrode and the second electrode comprises an AC potential.

14. The method of claim 10, wherein the resultant signal comprises an analyte signal representative of a concentration of the analyte at the first electrode.

## Patentansprüche

1. Vorrichtung umfassend:
ein Mikronadelarray (70) umfassend:
eine erste Mikronadel (30a) umfassend eine erste analytselektive Elektrode;
eine zweite Mikronadel (30b) umfassend eine zweite analytinvariante Elektrode (40b); und
ein selektives Erkennungselement (41), das an der ersten Elektrode angeordnet ist und dazu ausgelegt ist, eine erste elektrische Antwort zu erzeugen, die aus einer Wechselwirkung des selektiven Erkennungselements und eines Analyten entsteht; und
einen Prozessor,
wobei die erste Mikronadel und die zweite Mikronadel dazu ausgelegt sind, innerhalb einer lebensfähigen Epidermis oder einer Dermis eines Benutzers positioniert zu werden, wobei der Prozessor dazu ausgelegt ist, die erste elektrische Antwort, die eine erste Impedanz von der ersten Elektrode umfasst, und eine zweite elektrische Antwort, die eine zweite Impedanz von der zweiten Elektrode umfasst, zu messen, wobei die erste elektrische Antwort und die zweite elektrische Antwort aus dem Anlegen eines Vorspannungspotentials, das sowohl an die erste Elektrode als auch an die zweite Elektrode angelegt wird, folgen, und
wobei der Prozessor dazu ausgelegt ist, ein resultierendes Signal zu bestimmen, das die an der ersten Elektrode erzeugte erste elektrische Antwort und die an der zweiten Elektrode erzeugte zweite elektrische Antwort auf der Basis eines Verhältnisses der ersten elektrischen Antwort und der zweiten elektrischen Antwort darstellt.

2. Vorrichtung nach Anspruch 1, wobei die erste Elektrode ferner eine erste Membran umfasst, die auf dem selektiven Erkennungselement angeordnet ist.

3. Vorrichtung nach Anspruch 2, wobei die zweite Elektrode eine zweite Membran umfasst, die auf der zweiten Elektrode angeordnet ist.

4. Vorrichtung nach Anspruch 3, wobei die erste Membran und die zweite Membran das gleiche Material umfassen.

5. Vorrichtung nach Anspruch 1, wobei die erste Impedanz eine erste frequenzabhängige Impedanz umfasst und die zweite Impedanz eine zweite frequenzabhängige Impedanz umfasst.

6. Vorrichtung nach Anspruch 1, wobei das an die erste Elektrode und an die zweite Elektrode angelegte Vorspannungspotential identisch ist.

7. Vorrichtung nach Anspruch 1, wobei das an die erste Elektrode und an die zweite Elektrode angelegte Vorspannungspotential ein AC-Potential umfasst.

8. Vorrichtung nach Anspruch 1, wobei das resultierende Signal ein Analytsignal umfasst, das eine Konzentration des Analyten an der ersten Elektrode darstellt.

9. Vorrichtung nach Anspruch 1, wobei jede von der ersten Elektrode und der zweiten Elektrode an einem distalen Ende der entsprechenden Mikronadel angeordnet sind.

10. Verfahren umfassend:
Bereitstellen einer ersten Mikronadel (30a), die eine erste Elektrode (40a) umfasst, und einer zweiten Mikronadel (30b), die eine zweite Elektrode (40b) umfasst, die jeweils dazu ausgelegt sind, innerhalb einer lebensfähigen Epidermis oder einer Dermis eines Benutzers positioniert zu werden, wobei ein selektives Erkennungselement (41) an der ersten Elektrode angeordnet ist und dazu ausgelegt ist, eine erste elektrische Antwort zu erzeugen, die aus einer Wechselwirkung des selektiven Erkennungselements und eines Analyten entsteht;
Anlegen eines Vorspannungspotentials an sjede von der ersten Elektrode und der zweiten Elektrode;
Messen der ersten elektrischen Antwort von der ersten Elektrode und einer zweiten elektrischen Antwort von der zweiten Elektrode, wobei die erste elektrische Antwort eine erste Impedanz von der ersten Elektrode umfasst und die zweite elektrische Antwort eine zweite Impedanz von der zweiten Elektrode umfasst; und
Bestimmen eines resultierenden Signals, das die erste elektrische Antwort, die an der ersten Elektrode erzeugt wird, und die zweite elektrische Antwort, die an der zweiten Elektrode erzeugt wird, auf der Basis eines Verhältnisses der ersten elektrischen Antwort und der zweiten elektrischen Antwort darstellt.

11. Verfahren nach Anspruch 10, wobei die erste Elektrode ferner eine erste Membran umfasst, die auf dem selektiven Erkennungselement angeordnet ist.

12. Verfahren nach Anspruch 10, wobei die zweite Elektrode eine zweite Membran umfasst, die auf der zweiten Elektrode angeordnet ist, und wobei die erste Membran und die zweite Membran das gleiche Material umfassen.

13. Verfahren nach Anspruch 10, wobei das an die erste Elektrode und an die zweite Elektrode angelegte Vorspannungspotential identisch ist oder das an die erste Elektrode und die zweite Elektrode angelegte Vorspannungspotential ein AC-Potential umfasst.

14. Verfahren nach Anspruch 10, wobei das resultierende Signal ein Analytsignal umfasst, das eine Konzentration des Analyten an der ersten Elektrode darstellt.

## Revendications

1. Dispositif comprenant :
une matrice de micro-aiguilles (70) comprenant :
une première micro-aiguille (30a) comprenant une première électrode sélective vis-à-vis de l'analyte ;
une seconde micro-aiguille (30b) comprenant une seconde électrode invariante vis-à-vis de l'analyte (40b) ; et
un élément de reconnaissance sélective (41) disposé sur la première électrode et configuré pour générer une première réponse électrique provenant d'une interaction de l'élément de reconnaissance sélective et d'un analyte ; et
un processeur,
dans lequel la première micro-aiguille et la seconde micro-aiguille sont configurées pour être positionnées dans un épiderme viable ou un derme d'un utilisateur,
dans lequel le processeur est configuré pour mesurer la première réponse électrique comprenant une première impédance de la première électrode et une seconde réponse électrique comprenant une seconde impédance de la seconde électrode, la première réponse électrique et la seconde réponse électrique résultant de l'application d'un potentiel de polarisation appliqué sur chacune de la première électrode et de la seconde électrode, et
dans lequel le processeur est configuré pour déterminer un signal résultant représentatif de la première réponse électrique générée au niveau de la première électrode et de la seconde réponse électrique générée au niveau de la seconde électrode sur la base d'un rapport entre la première réponse électrique et la seconde réponse électrique.

2. Dispositif selon la revendication 1, dans lequel la première électrode comprend en outre une première membrane disposée sur l'élément de reconnaissance sélective.

3. Dispositif selon la revendication 2, dans lequel la seconde électrode comprend une seconde membrane disposée sur la seconde électrode.

4. Dispositif selon la revendication 3, dans lequel la première membrane et la seconde membrane comprennent le même matériau.

5. Dispositif selon la revendication 1, dans lequel la première impédance comprend une première impédance dépendant de la fréquence et la seconde impédance comprend une seconde impédance dépendant de la fréquence.

6. Dispositif selon la revendication 1, dans lequel le potentiel de polarisation appliqué à la première électrode et à la seconde électrode est identique.

7. Dispositif selon la revendication 1, dans lequel le potentiel de polarisation appliqué à la première électrode et à la seconde électrode comprend un potentiel de courant alternatif.

8. Dispositif selon la revendication 1, dans lequel le signal résultant comprend un signal d'analyte représentatif d'une concentration de l'analyte au niveau de la première électrode.

9. Dispositif selon la revendication 1, dans lequel chacune de la première électrode et de la seconde électrode est disposée à une extrémité distale de la micro-aiguille respective.

10. Procédé comprenant :
la fourniture d'une première micro-aiguille (30a) comprenant une première électrode (40a) et d'une seconde micro-aiguille (30b) comprenant une seconde électrode (40b) configurées chacune pour être positionnées au sein d'un épiderme viable ou d'un derme d'un utilisateur, dans lequel un élément de reconnaissance sélective (41) est disposé sur la première électrode et configuré pour générer une première réponse électrique provenant d'une interaction de l'élément de reconnaissance sélective et d'un analyte ;
l'application d'un potentiel de polarisation sur chacune de la première électrode et de la seconde électrode ;
la mesure de la première réponse électrique de la première électrode et d'une seconde réponse électrique de la seconde électrode, la première réponse électrique comprenant une première impédance de la première électrode et la seconde réponse électrique comprenant une seconde impédance de la seconde électrode ; et
la détermination d'un signal résultant représentatif de la première réponse électrique générée au niveau de la première électrode et de la seconde réponse électrique générée au niveau de la seconde électrode sur la base d'un rapport entre la première réponse électrique et la seconde réponse électrique.

11. Procédé selon la revendication 10, dans lequel la première électrode comprend en outre une première membrane disposée sur l'élément de reconnaissance sélective.

12. Procédé selon la revendication 10, dans lequel la seconde électrode comprend une seconde membrane disposée sur la seconde électrode, et dans lequel la première membrane et la seconde membrane comprennent le même matériau.

13. Procédé selon la revendication 10, dans lequel le potentiel de polarisation appliqué à la première électrode et à la seconde électrode est identique ou le potentiel de polarisation appliqué à la première électrode et à la seconde électrode comprend un potentiel de courant alternatif.

14. Procédé selon la revendication 10, dans lequel le signal résultant comprend un signal d'analyte représentatif d'une concentration de l'analyte au niveau de la première électrode.
